# FASCICULE DE BREVET EUROPEEN

(11) **EP 0 804 148 B1**
(45) Date de publication et mention de la délivrance du brevet: **08.03.2006**
(21) Numéro de dépôt: 95926412.8
(22) Date de dépôt: 21.07.1995
(51) Int. Cl.: A61K 8/365, A61K 8/37, A61K 8/42, A61Q 19/00

(54) **ACIDE HYDROXYLE LIPOPHILE, SON UTILISATION EN COSMETIQUE ET EN PHARMACIE, ET SON PROCEDE DE PREPARATION**
LIPOPHILE HYDROXYSÄURE, UND IHRE VERWENDUNG IN DER KOSMETIK UND DER PHARMAZIE UND VERFAHREN ZUR HERSTELLUNG
LIPOPHILIC HYDROXYLATED ACID, COSMETIC AND PHARMACEUTICAL USE THEREOF AND METHOD FOR PREPARING SAME

(30) Priorité: 22.07.1994 FR 9409091
(43) Date de publication de la demande: 05.11.1997
(73) Titulaire: ENGELHARD LYON, 69007 Lyon (FR)
(72) Inventeur: PERRIER, Eric, F-38200 Vienne (FR); ANTONI, Danièle, F-69390 Vernaison (FR); HUC, Alain, F-69110 Sainte-Foy-lès-Lyon (FR)
(74) Mandataire: Portal, Gérard
(86) Numéro de dépôt international: PCT/FR1995/000984
(87) Numéro de publication internationale: WO 1996/003110

(56) Documents cités:
- EP-A- 0 338 565
- EP-A- 0 447 064
- EP-A- 0 514 067
- EP-A- 0 526 302
- DE-A- 1 543 929

## Description

La présente invention concerne essentiellement un nouvel acide hydroxylé lipophile, son utilisation en cosmétique et en pharmacie, ainsi que son procédé de préparation.

Il est connu par l'article de Van Scott et al. dans Arch. Dermatol. Vol. 110, octobre 1974, pages 586-590 d'utiliser les acides α-hydroxylés dans des préparations topiques pour effectuer une kératolyse particulièrement efficace dans le traitement de l'ichthyose et des peaux sèches.

De même, Middleton a décrit dans J. Soc. Cosmet. Chem. 25 (1974), pages 519-534, une crème pour la peau contenant de l'acide lactique ou du lactate de sodium pour réduire la peau sèche formant des écailles au niveau de la couche cornée ou Stratum corneum.

Encore, il a été publié par Malcolm W. Greaves dans Cosmctics et Toiletries, Vol. 105, d'octobre 1990, pages 61-64, l'emploi de dérivés d'acide α-hydroxylés, par emploi topique, pour traiter la peau sèche associée à de l'eczéma. Cet article souligne en page 61, colonne de droite, avant-dernier paragraphe, que comme l'urée, les acides α-hydroxylés ont été d'un emploi limité dû à leur caractère irritant. Cet article concerne l'emploi d'une composition contenant un méthoxypropylgluconamide ayant une plus faible acidité.. Ce composé fait également l'objet d'une demande de brevet européen EP-A-0 338 565 de REVLON. Cette demande européenne concerne un composé de formule : 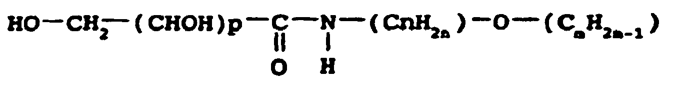 formule dans laquelle p est un nombre entier de 1 à 4,
(CₙH₂ₙ) est un pont alkyle à chaîne droite ou ramifiée dans laquelle n est un nombre entier de 1 à 6, et (CₘH₂ₘ₋₁) est un groupe alkyle à chaîne droite ou ramifiée dans laquelle m est un nombre entier de 1 à 6.

Ces composés sont donc des dérivés amides d'un acide au moins dihydroxylé, et qui comportent obligatoirement une chaîne éther, composés qui sont différents de ceux mis au point et utilisés dans le cadre de la présente invention.

Greaves et al. ont observé que les composés décrits dans ce document EP-A-0 338 565 sont peu efficaces en tant qu'agents kératolytiques d'où la nécessité de les utiliser à des concentrations très élevées. En outre, cette propriété kératolytique n'est pas modulable car très semblable d'un produit à l'autre.

Par contre, dans le cadre de la présente invention, il a pu être découvert de nouveaux composés dont l'activité peut être modulable depuis une activité très puissante jusqu'à une activité beaucoup plus douce.

Le document EP-A-0 273 202, Van Scott et al. décrit des additifs, à base d'acide hydroxycarboxylique, favorisant les actions topiques d'agents thérapeutiques, présent en une quantité allant de 0,01 à 99% en poids de la composition totale.

Les acides α-hydroxylés, leurs sels et leur forme lactone décrits dans le document EP-A-0 273 202 ont un pouvoir kératolytique très fort lorsqu'ils sont utilisés à de fortes concentrations et à des pH acides. Cependant, dans de telles conditions d'utilisation, ils sont mal tolérés par la surface cutanée et provoquent picotement, rougeur et phénomènes inflammatoires, ce qui limite très fortement leur utilisation dans de telles conditions.

Dans la présente invention, il a été découvert de nouveaux produits qui sont non-irritants, dans un large domaine de pH pouvant aller d'un pH très acide voisin de 3 ou 3,5 à un pH neutre de l'ordre de 7, cela même lorsqu'ils sont testés à des concentrations très importantes comme 100 %.

Il est aussi connu par les documents VAN SCOTT US 4 105 783 et 4197 316 des amides d'acide hydroxylé obtenus avec une monoamine ou une diamine pouvant notamment comporter un radical alkyle en C1-C8. Dans le cadre de l'invention, lorsqu'il s'agit d'amides, ceux-ci sont obtenus avec une monoamine ayant un radical alkyle gras en C10-C30.

Le document REVLON EP-A-0 447 064 est encore relatif à des alcoxyamides du type de ceux décrits dans le document précédent EP-A-0 338 565 qui sont différents de ceux mis au point et utilisés dans le cadre de la présente invention.

Divers autres documents sont relatifs à des esters de la fonction acide d'acide hydroxylé qui sont différents de ceux mis au point et utilisés dans le cadre de la présente invention. Ces documents relatifs à des esters sont WO-A-95/03032, EP-A-0 599 819 VAN SCOTT, EP-A-0 273 202 ; EP-A-0 521 647 UNILEVER dans lequel on décrit des esters d'acide gras d'acide citrique dont les fonctions acides de l'acide citrique sont obligatoirement substituées par des liaisons esters avec un groupe de substition ester R1, R2 et R3. La fonction hydroxy de l'acide citrique peut être substituée par un groupement acyle R4 qui est de préférence un groupe acyle ayant de 2 à 4 atomes de carbone. Dans le cadre de l'invention, des liaisons esters sur le groupement acide de l'acide hydroxylé ne sont pas recherchées et une liaison ester sur la fonction hydroxy de l'acide hydroxylé comprend au minimum 7 atomes de carbone.

La demande de l'OREAL EP-A-0 526 302 est relative à des sels ou esters d'acide 2,5-dihydroxyphénylcarboxylique dont les esters sont réalisés sur la fonction acide qui sont différents de ceux mis au point et utilisés dans le cadre de la présente invention. Comme autres esters de la fonction acide de l'acide hydroxylé, on peut citer les documents UNILEVER Ep-A-0 282 289 ; EP-A-0261 812 ; le document US-A-5 302 377 de CRODA ; le document US-A-4 078 147 UKAI ; les brevets suisses CH-A-449 852 et 443 565 des LABORATOIRES PROD'HYG, le brevet français HENKEL FR-A-2 390 160 ; le document HENKEL DE-4 033 565.

Comme amide, on citera encore le document allemand DE-A-1543 929 qui décrit des amides de l'acide 2,4-dihydroxy-3,3-diméthylbutyrique à partir d'une hydroxyalkylamine ou d'une alcoxyalkylamine en C5 à C30, soit une aminé différente des alkylamines de l'invention ayant de C10-C30.

Par ailleurs, il est aussi connu par le document FR-A-2 581 542 t'Oreal, des compositions topiques destinées au traitement de la peau, à base de dérivés de l'acide salicylique. Ces dérivés répondent à la formule : 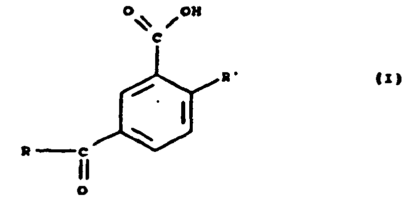 dans laquelle R représente une chaîne hydrocarbonée pouvant avoir jusqu'à 17 atomes de carbone et R' pouvant représenter une fonction hydroxyle ou une fonction ester de formule 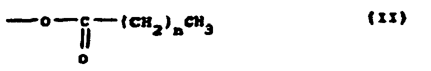 comportant de 2 à 16 atomes de carbone, cette chaîne pouvant être saturée ou insaturée, n pouvant avoir une valeur comprise entre 0 et 14 (voir la description et la revendication 1 en particulier).

Les composés préférés sont ceux pour lesquels R' désigne une fonction hydroxyle et R un groupement alkyle ayant de 3 à 11 atomes de carbone (revendication 2 et page 3, lignes 10 à 20).

Il ressort de cette formule que ces dérivés sont obligatoirement substitués en position 5 du noyau phényl par un radical comportant une fonction cétone qui apparaît être déterminante pour fournir une activité kératolytique supérieure à celle de l'acide salicylique, comme indiqué dans la description en page 2, lignes 5 à 10.

Dans le cadre de la présente invention, on a découvert de nouveaux dérivés d'acide hydroxylé particulièrement actifs et non-irritants, incluant des dérivés de l'acide salicylique différents des dérivés substitués en position 5 du noyau phényle de l'acide salicylique décrits dans ce document.

En outre, il a été découvert que les dérivés de l'invention présentaient également un pouvoir émulsionnant important permettant leur utilisation comme agent émulsionnant unique, c'est-à-dire utilisé seul, ou comme agent co-émulsionnant permettant de diminuer la quantité d'autres agents émulsionnants.

Par ailleurs, les dérivés 5-céto-substitués de l'acide salicylique décrites dans le document précèdent FR-A-2 581542 présentent l'inconvénient majeur que la liaison cétone n'est pas hydrolysable par des voies enzymatiques. En outre, bien qu'une fonction ester soit prévue sur la fonction hydroxyle le seul dérivé préparé est l'ester d'acétyle dans l'exemple de préparation G dont il apparaît d'après les essais de pharmacologie des tableaux I et II, qu'il est moins actif que les dérivés libres.

En fait, ce document FR-A-2581 542 enseigne à l'homme de l'art la nécessité d'utiliser des dérivés substitués en position 5 de l'acide salicylique par une liaison cétone et le fait que la fonction hydroxyle de l'acide salicylique doit être libre.

Ceci est confirmé par le document FR-A-2 607 498 de l'Oréal qui concerne des salicylates lipophiles d'ammonium quaternaire dans lesquels la fonction hydroxyle de l'acide salicylique est non substituée et qui comporte toujours en position 5 le radical lié par une liaison cétone au cycle phényle, ce salicylate étant sous forme ionique grâce à la présence d'un ammonium quaternaire venant se coupler à la fonction COO- de l'acide. En conséquence, ces documents dissuadent l'homme de l'art de rechercher des dérivés d'acide salicylique autres que les dérivés 5-céto-substitués c'est-à-dire portant un radical lié par une liaison cétone.

Ces composés présentent cependant l'inconvénient qu'ils ne sont pas hydrolysables soit spontanément, soit sous l'action d'enzymes cutanées ou bactériennes.

Le document EP-A-0 378 936 utilise encore les mêmes dérivés de l'acide salicylique pour réaliser le traitement du vieillissement de la peau, qui présentent les mêmes inconvénients de n'être pas hydrolysables soit spontanément, soit sous l'action d'enzymes cutanées ou bactériennes.

Par ailleurs, le document EP-A-0 433 104 Unilever est relatif à une composition de shampooing contenant l'acide 2-hydroxyalcanoïque en combinaison avec un agent tampon formant un co-acide, de sorte que le pH de la composition est de préférence compris entre 3 et 5.

Encore, le document EP-A-0 413 528 de Yu et Van Scott est relatif à des compositions amphotères et des formes polymères d'α-hydroxyacide et leur utilisation thérapeutique pour traiter les peaux sèches, l'acnée, les kératoses, le psoriasis, l'eczéma, les taches de vieillissement, les rides, la peau blême, la peau hyperpigmentee, la peau hyperkératinisée, les dermatoses inflammatoires, les changements de peau associés avec l'âge et en tant que produit nettoyant de la peau. En fait, comme il ressort de la liste donnée en pages 29 et 30, il s'agit des mêmes hydroxyacides que ceux qui sont décrits dans le document EP-A-0 273 202 précédemment analysé. Ces produits sont mal tolérés par la surface cutanée et provoquent picotements, rougeurs et phénomènes inflammatoires limitant très fortement leur utilisation.

Un document similaire est encore constitué par le document EP-A-0 508 324 déposé par Yu et Van Scott pour traiter les signes de vieillissement de la peau, des ongles et des cheveux.

Enfin, le document EP-A-0 585 170 est encore un document de l'Oréal relatif à une composition pour le traitement de l'acné contenant le sel d'ammonium quaternaire du dérivé 5-cétone de l'acide salicylique avec un ion ammonium quaternaire, encapsulé dans des vésicules de type liposome.

Il est encore connu par le document EP-A-0 526 302 l'OREAL une composition à action dépigmentante à base de dérivés d'acide (2,5-dihydroxyphényl)carboxylique. Ces composés sont exclus dans le cadre de la présente invention en tant que produit nouveau et également dans le cadre d'une action dépigmentante.

Egalement, le document EP-A-0 514 067 UNILEVER est relatif à une composition cosmétique appropriée pour une application topique sur la peau humaine pour réduire les effets néfastes de la lumière ultraviolette sur la peau, comprenant une quantité efficace d'un triester d'acide citrique. Les triesters d'acide citrique sont exclus des nouveaux composés, ainsi que d'une utilisation dépigmentante ou ayant un effet antivieillissement sur la peau.

On constate ainsi que l'art antérieur démontre que des recherches intensives sont réalisées concernant l'emploi d'acides α-hydroxylés dans des compositions cosmétique ou pharmaceutique mais qu'aucune des solutions proposées n'a été capable de résoudre simultanément le problème de la tolérance par la surface cutanée de ces produits, les acides étant trop nocifs, et le problème de leur efficacité modulable ainsi que de leur biocompatibilité en étant dégradable ou hydrolysable spontanément ou sous l'action d'enzymes cutanées ou bactériennes. En outre, leur affinité vis-à-vis des constituants lipidiques de l'épiderme est restée limitée.

La présente invention a pour but de résoudre le nouveau problème technique consistant en la fourniture de nouveaux produits utilisables comme produits cosmétiques ou pharmaceutiques et/ou dermatologiques présentant une affinité plus grande vis-à-vis des constituants lipidiques de l'épiderme, en particulier du stratum corneum, non irritants et présentant une efficacité modulable.

La présente invention a aussi pour but principal de résoudre le nouveau problème technique consistant en la fourniture de nouveaux produits utilisables comme produits cosmétiques ou pharmaceutiques et/ou dermatologiques présentant une excellente biocompatiblité, notamment en étant hydrolysables soit spontanément, soit sous l'action d'enzymes cutanées ou bactériennes.

La présente invention a aussi pour but de résoudre ces nouveaux problèmes techniques selon une solution qui permette de fournir des composés présentant une bonne activité kératolytique en étant capables d'atteindre les desmosomes ainsi que les couches naissantes du stratum corneum.

La présente invention a encore pour but de résoudre le nouveau problème technique consistant en la fourniture d'une solution qui permette de fournir des produits ayant une activité anti-acnéique, et/ou anti-verrues, et/ou anti-eczéma, et/ou anti-psoriasis, et/ou anti-pelliculaire, et/ou anti-peau sèche, et/ou anti-rides et/ou anti-âge, sans pouvoir irritant notable.

La présente invention a encore pour but de résoudre le nouveau problème technique consistant en la fourniture d'une solution permettant de réaliser de nouveaux agents améliorant l'hydratation, l'élasticité, la cohésion de la peau, ainsi que de nouveaux agents dépigmentants, sans pouvoir irritant notable.

La présente invention a encore pour but principal de résoudre le nouveau problème technique consistant en la fourniture d'une solution permettant de réaliser de nouveaux produits qui présentent une excellente capacité émulsion- nante permettant de les utiliser soit comme émulsionnant unique, soit comme co-émulsionnant en permettant ainsi de diminuer la concentration d'autres agents émulsionnants utilisés, en particulier lors de la fabrication de compositions cosmétiques ou pharmaceutiques et/ou dermatologiques.

Il a été découvert de manière totalement inattendue que l'ensemble de ces problèmes techniques pouvait être résolu par la fourniture de nouveaux dérivés d'acides hydroxylés, décrits ci-après. Il a également été découvert que l'efficacité de ces nouveaux dérivés était accrue lorsque ces dérivés sont utilisés en étant présents dans le milieu réactionnel final avec les acides de départ n'ayant pas réagi.

Ainsi, selon un premier aspect, la présente invention fournit de nouveaux acides hydroxylés lipophiles, lesdits acides hydroxylés comprenant au moins une fonction hydroxyle et au moins une fonction acide, caractérisés en ce que ledit acide hydroxylé, lipophile, comprend une chaîne hydrocarbonée hydrophobe ayant de 7 à 30 atomes de carbone greffée sur la fonction hydroxyle et/ou la fonction acide de l'acide hydroxylé lipophile par une liaison covalente choisie parmi le groupe consistant d'une liaison ester sur la fonction hydroxyle, d'une liaison anhydride sur la fonction acide et d'une liaison amide sur la fonction acide à la condition que dans le cas d'une liaison amide cette chaîne hydrocarbonée hydrophobe soit constituée par un radical alkyle ayant de 10 à 30 atomes de carbone résultant d'une monoamine, ou d'une combinaison de ces liaisons et ledit acide hydroxylé avant greffage de la chaîne hydrocarbonée hydrophobe est choisi parmi le groupe consistant d'un acide alpha-hydroxylé, choisi parmi l'acide glycolique, l'acide lactique, l'acide malique, l'acide tartrique, l'acide gluconique, l'acide méthyl lactique, l'acide mandélique, l'acide atrolactique, l'acide phényl lactique, l'acide glycérique, l'acide benzylique, l'acide alpha-hydroxyhexanoique, l'acide alpha-hydroxyheptanoique, l'acide alpha-hydroxyoctanoique ; ou un acide bêta-hydroxylé, choisi parmi l'acide salicylique, la sérine ; un polymère ou un copolymère de cet acide hydroxylé, ou leurs mélanges ;

Selon un premier mode de réalisation, cet acide hydroxylé lipophile est caractérisé en ce que l'acide hydroxylé avant greffage de la chaîne hydrocarbonée hydrophobe précitée comprend une chaîne droite ou ramifiée, ou cyclique ayant de 2 à 30 atomes de carbone, saturée ou insaturée.

Selon un autre mode de réalisation, cet acide hydroxylé lipophile est caractérisé en ce que le greffage de la chaîne hydrocarbonée hydrophobe précitée est réalisé par réaction d'un acide hydroxylé avec une amine, un acide ou un alcool, ledit acide, ledit alcool ou ladite amine comprenant ladite chaîne hydrocarbonée hydrophobe qui, de préférence, comprend de 2 à 30 atomes de carbone, ladite chaîne hydrocarbonée hydrophobe pouvant être saturée ou insaturée, linéaire ou ramifiée, ou cyclique sans autre fonction sur son squelette, ou avec d'autres fonctions, en particulier acide(s), alcool(s) et/ou amine(s).

Selon encore un autre mode de réalisation, cet acide hydroxylé lipophile est caractérisé en ce que ledit acide hydroxylé est mono- ou polyhydroxylé et est un monoacide ou un polyacide.

Selon un autre mode de réalisation, cet acide hydroxylé liphophile est caractérisé en ce que l'acide hydroxylé avant greffage de la chaîne hydrocarbonée hydrophobe comprend de 2 à 18 atomes de carbone.

Naturellement, comme cela est bien compréhensible à l'homme de l'art, cette liste n'est pas du tout exhaustive puisque l'ensemble des acides hydroxylés, peut être utilisé dans le cadre de l'invention. Cest encore un avantage de l'invention que des acides ayant une fonction hydroxy plus éloignée de la fonction acide puissent être utilisés avantageusement. L'invention couvre ainsi l'emploi de tout acide hydroxylé sans limitation. Cependant, les acides hydroxylés actuel le ment préférés sont les acides qui ont une chaîne hydrocarbonée initiale de 2 à 30 atomes de carbone, et encore de préférence de 2 à 18 atomes de carbone.

Selon un autre mode de réalisation, cet acide hydroxylé lipophile est caractérisé par un rapport en poids entre l'acide hydroxylé et la chaîne hydrophobe de 0,05 à 10 et de préférence de 0,05 à 2.

Selon un deuxième aspect, la présente invention concerne également l'utilisation de l'acide hydroxylé lipophile précité :
- soit comme agent cosmétique ou comme principe actif pour la fabrication d'une composition cosmétique,
- soit pour la fabrication d'une composition dermatologique et/ou pharmaceutique,
- soit comme agent émulsionnant.
   Selon un mode de réalisation particulier, l'utilisation est caractérisée en ce que les acides hydroxylés lipophiles précités, en tant que principe actif, présentent une activité kératolytique, une activité de gommage chimique en profondeur de la peau, permettant d'améliorer la pénétration ultérieure d'autres ingrédients actifs, une activité stimulatrice des fonctions cellulaires, améliorant l'élasticité, la cohésion de la peau, une activité dépigmentante, une activité antirides ou anti-âge, une activité hydratante permettant le traitement des peaux sèches et ichtyosiques, une activité anti-acnéique, anti-verrues, anti-eczéma, anti-psoriasis, anti-pelliculaire.
   Selon encore un autre mode de réalisation particulier, l'utilisation est caractérisée en ce que les acides hydroxylés lipophiles précités constituent des agents émulsionnants particulièrement intéressants, qu'ils soient utilisés en tant qu'agent émulsionnant unique, ou en tant qu'agent co-émulsionnant. Par ailleurs, lorsqu'ils sont utilisés comme principe actif à des concentrations relativement fortes, en raison de leur activité émulsionnante, il n'est pas nécessaire d'utiliser d'autres agents émulsionnants, ce qui constitue un effet technique inattendu, particulièrement intéressant dans la formulation de la composition cosmétique, pharmaceutique et/ou dermatologique dans la mesure où les agents émulsionnants habituels présentent toujours des risques de bio-incompatibilité.
   Selon un troisième aspect, la présente invention couvre également une composition choisie parmi le groupe consistant en une composition cosmétique, pharmaceutique et/ou dermatologique, caractérisée en ce qu'elle comprend comme ingrédient actif et/ou comme agent émulsionnant au moins un acide hydroxylé lipophile précité.
   Selon un mode de réalisation particulier, la composition est
caractérisée en ce que la proportion d'acide hydroxylé lipophile est comprise entre 0,001 % et 50 % en poids de la composition finale, de préférence entre 1 % et 20 % en poids de la composition finale.

Selon un autre mode de réalisation particulier, la composition est caractérisée en ce que l'acide hydroxylé lipophile précité est le produit de réaction d'un acide hydroxylé choisi parmi le groupe consistant de l'acide malique, de l'acide glycolique, de l'acide gluconique, de l'acide salicylique, de l'acide lactique, de la sérine, d'un polymère de l'acide glycolique, d'un polymère de l'acide lactique, d'un copolymère de l'acide glycolique et de l'acide lactique ; et d'un composé comprenant la chaîne hydrocarbonée hydrophobe précitée choisi parmi le groupe consistant d'un halogénure ou anhydride d'acide stéarique, d'acide palmitique, d'acide myristique, d'acide laurique, d'acide octanoïque, d'acide décanoïque, d'acide undécylénique, d'acide undécanoïque, d'acide oléique, d'acide linolénique, d'acide linoléique, d'acide acétique, ou de leurs dérivés succiniques ou maléiques.

Selon un autre mode de réalisation, la composition est caractérisée en ce que l'acide hydroxylé lipophile précité est le produit de réaction d'un acide hydroxylé avec une monoamine à radical alkyle à chaîne droite, ramifiée ou cyclique ayant de 10 à 30 atomes de carbone.

Selon un quatrième aspect, la présente invention couvre encore un procédé de fabrication d'acide hydroxylé lipophile précité, caractérisé en ce qu'il comprend la réaction d'un acide hydroxylé avec un halogénure ou un anhydride d'un acide comprenant une chaîne hydrocarbonée hydrophobe ayant de 2 à 30 atomes de carbone, ou avec une monoamine à radical alkyle à chaîne droite, ramifée ou cyclique ayant de 10 à 30 atomes de carbone.

Selon un mode de réalisation particulier, le procédé est caractérisé en ce que l'acide hydroxylé ou l'halogénure ou l'anhydride d'acide, ou la monoamine précités sont tels que définis précédemment.

Selon un autre mode de réalisation particulier, le procédé de fabrication est caractérisé en ce que pour le greffage d'une chaîne hydrocarbonée hydrophobe provenant d'un acide, on utilise comme produit de départ un halogénure ou un anhydride de l'acide.

Selon un autre mode de réalisation particulier, le procédé est caractérisé en ce que pour le greffage d'une chaîne hydrocarbonée hydrophobe provenant d'une monoamine, on utilise comme produit de départ une monoamine qui est mise à réagir avec l'acide hydroxylé en présence d'un catalyseur, d'un agent bifonctionnel, ou d'une enzyme.

Cet agent bifonctionnel est bien connu à l'homme de l'art. Des agents bifonctionnels particulièrement préférés sont un carbodiimide, en particulier le chlorhydrate de N-(diméthylamino-3-propyl)-N'-éthylcarbodiimide, le chlorure d'oxalyle, le diphénylphosphorylazide, l'anhydride propane phosphonique, ou tout autre agent couramment utilisé comme agent de couplage lors des synthèses linéaires peptidiques.

Selon encore un autre mode de réalisation particulier, le procédé est caractérisé en ce qu'on réalise un greffage enzymatique d'une chaîne hydrocarbonée hydrophobe sur l'acide hydroxylé. De telles enzymes capables de réaliser ce greffage sont bien connues à l'homme de l'art. Par exemple, on pourra citer une lipase industrielle, par exemple l'enzyme disponible dans le commerce sous la marque Lipozyme® fabriquée par la société Novo®. Ce greffage enzymatique est particulièrement intéressant pour le greffage d'amines.

Selon un autre mode de réalisation particulier, le procédé est caractérisé en ce que la réaction a lieu à une température comprise entre environ 0°C et environ 100°C, de préférence à la température ambiante, en milieu aqueux ou solvant organique.

Il est à noter que les réactions de greffage peuvent être réalisées à température élevée (entre 80° et 100°C) pour donner des rendements plus importants mais impliquent plus de contraintes et des risques de dégradation des produits.

Par ailleurs, les proportions relatives entre l'acide hydroxylé et la substance apportant la chaîne hydrocarbonée hydrophobe peuvent varier dans de larges limites. Ainsi, les proportions relatives en poids, utilisées lors de la réaction, peuvent varier de 5/93 à 95/5, de préférence de 5/95 à 50/50.

D'autre part, il peut être avantageux de neutraliser tout d'abord l'acide hydroxylé à un pH voisin de la neutralité, c'est-à-dire voisin de 7. On peut également prévoir de maintenir le pH de la réaction à une valeur basique au cours de la réaction.

Dans ce cas, il est possible d'ajuster le pH en continu soit à l'aide d'une base forte du type NaOH ou KOH, ou de prévoir un tampon tel que phosphate, carbonate, borate ou citrate ou l'ajout d'une molécule adaptée pour le piégeage en continu de l'acide fabriqué au cours de la réaction tel que triéthanolamine (TEA), cyclohexylamine, etc...

Par ailleurs, après la réaction, il peut être également avantageux d'ajuster le pH en fonction des applications envisagées, généralement entre environ 2 et environ 7. Il peut aussi être avantageux de lyophiliser le milieu réactionnel final pour obtenir un produit lyophilisé contenant l'ensemble des produits du milieu réactionnel, à savoir l'acide hydroxylé lipophile mélangé au(x) produit(s) de départ n'ayant pas réagi, en particulier les acides et/ou monoamines, ce qui est particulièrement avantageux dans le cadre de l'invention.

On peut éventuellement réaliser de manière classique une stérilisation surtout dans le cadre d'un emploi cosmétique, pharmaceutique ou dermatologique, par exemple par des rayons beta ou gamma.

Grâce à l'invention, on obtient de nouveaux acides hydroxylés lipophiles qui possèdent une affinité plus grande vis-à-vis des constituants lipophiles du stratum corneum et sont donc capables de s'intégrer plus facilement dans toute l'épaisseur du stratum corneum.

En outre, grâce au greffage d'un groupe lipophile sur les acides hydroxylés, via des liaisons esters sur la fonction hydroxyle et/ou des liaisons amides et anhydrides sur la fonction acide de l'acide hydroxylé, on utilise des liaisons hydrolysables par des systèmes enzymatiques naturellement présents dans la peau, tels que des lipases et des protéases ainsi qu'à la surface de celle-ci telles que des enzymes libérées par les micro-organismes saprophytes présents en très grand nombre sur la peau.

Ainsi, les nouveaux composés de l'invention permettent par une hydrolyse naturelle de libérer progressivement les acides hydroxylés, ce qui permet d'obtenir la mise en oeuvre de leur activité sur le site où l'acide hydroxylé aura été véhiculé ou transporté par le greffage lipophile et d'obtenir un effet retard en maintenant cette activité sur de longues durées, ce qui permet de diminuer de manière inattendue les concentrations d'utilisation.

Il a également pu être observé que les produits selon l'invention sont essentiellement non irritants, contrairement aux acides hydroxylés antérieurement utilisés.

Les produits de l'invention présentent également de nouvelles activités qui ont été précédemment énoncées.

En particulier, ces produits sont des agents émulsionnants, particulièrement efficaces. Ainsi, l'invention couvre également une composition comprenant comme agent émulsionnant au moins un acide hydroxylé lipophile, en particulier à une concentration comprise entre 0,1 % et 50 % en poids de la composition finale, de préférence entre 1 et 20 % en poids de la composition finale.

Dans le cadre de l'invention, il est préféré que les produits ne soient pas purifiés, c'est-à-dire qu'ils soient présents dans le milieu réactionnel comprenant les acides de départ n'ayant pas réagi, ce qui signifie qu'une partie seulement (de 5 à 90 % en moles, et le plus souvent 10 à 30 % en moles) des acides hydroxylés sont transformés en esters, en amides ou en anhydrides, l'autre partie étant complexée d'une manière plus ou moins intense aux chaînes hydrophobes, via des liaisons ioniques, des liaisons du type Van der Waals, des liaisons hydrophiles et des liaisons hydrophobes. Dans le cas où le procédé de préparation implique l'utilisation d'un solvant non aqueux, ce solvant non aqueux étant en général non compatible avec une utilisation en cosmétique, pharmacie et/ou dermatologie, ce solvant sera habituellement éliminé.

Ils entrent ensuite, à l'état pur, ou de préférence à l'état brut de fabrication, c'est-à-dire dans le milieu réactif final comprenant les acides de départ n'ayant pas réagi, dans les formulations de préparations cosmétiques, pharmaceutiques et/ou dermatologiques.

Les acides hydroxylés lipophiles selon l'invention peuvent avantageusement être présentés sous une forme sèche, en particulier lyophilisée.

Dans le cadre de la présente description et des revendications, les termes "monoamine à radical alkyle ayant de 10 à 30 atomes de carbone concernent une amine comprenant une fonction amine unique, monosubstituée ou disubstituée par un radical alkyle ayant de 10 à 30 atomes de carbone, c'est-à-dire présentant une chaîne grasse, linéaire, ramifiée ou cyclique. De telles monoamines à chaîne grasse sont bien connues à l'homme de l'art et on citera à titre d'exemple la laurylamine ou la stéarylamine.

Selon un cinquième aspect, la présente invention concerne également une méthode cosmétique de traitement kératolytique, de zone(s) de la peau en ayant besoin, comprenant l'application auxdites zones de la peau d'une quantité efficace kératolytique d'au moins un composé acide hydroxylé lipophiletel que précédemment défini.

Des modes de réalisation préférés de la méthode sont clairement apparents pour un homme de l'art à partir de la description prise dans son ensemble et incluant l'ensemble des revendications qui sont incorporées par référence.

Selon un sixième aspect, l'invention concerne également une méthode cosmétique de traitement d'exfoliation chimique de la peau, pour stimuler les cellules de la peau, pour améliorer l'élasticité et la cohésion de la peau, pour dépigmenter la peau, pour hydrater la peau, pour réaliser un effet anti-rides de la peau, un effet anti-vieillissement sur la peau, un effet anti-pelliculaire sur des zones choisies des cheveux et du cuir chevelu, caractérisée en ce qu'elle comprend l'application sur la peau, la chevelure, le cuir chevelu en ayant besoin d'une quantité efficace d'un acide hydroxylé lipophile, tel que précemment défini. De manière similaire, des modes de réalisation préférés de la méthode sont clairement apparents à l'homme de l'art à partir de la description prise dans son ensemble et incluant l'ensemble des revendications qui sont incorporées par référence.

D'autres buts, caractéristiques et avantages de l'invention apparaîtront clairement à la lumière de la description explicative qui va suivre, faite en référence aux exemples ci-après, donnés simplement à titre d'illustration et qui ne sauraient en aucune façon limiter la portée de l'invention. Cependant, il est à observer que ces exemples contiennent clairement pour l'homme de l'art des données générales et permettent en conséquence de supporter des revendications de portée générale. Dans tous les exemples, les pourcentages sont donnés en poids, sauf indication contraire.

### Description des dessins

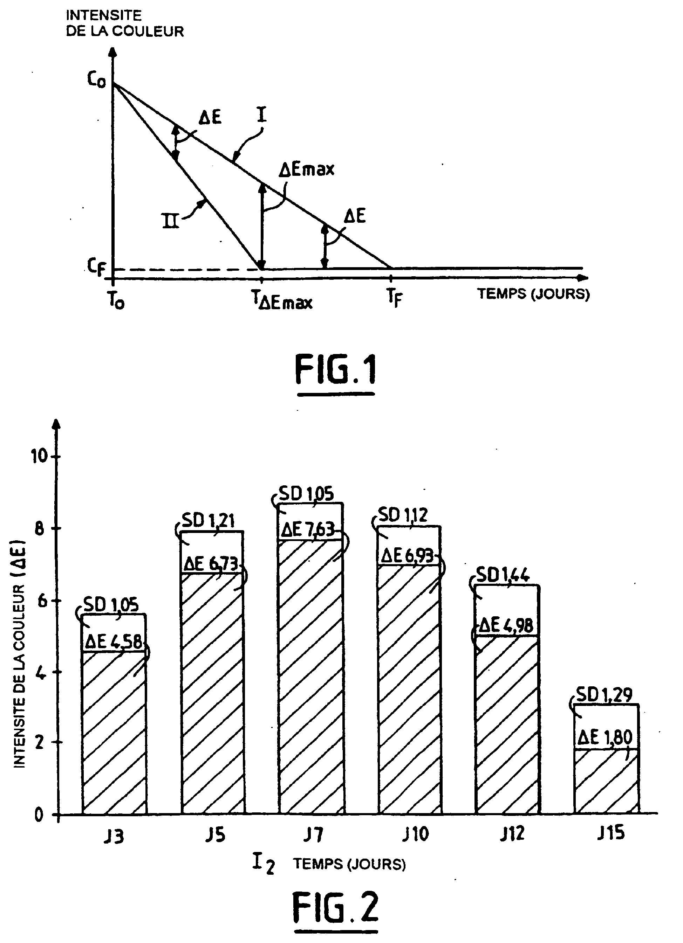

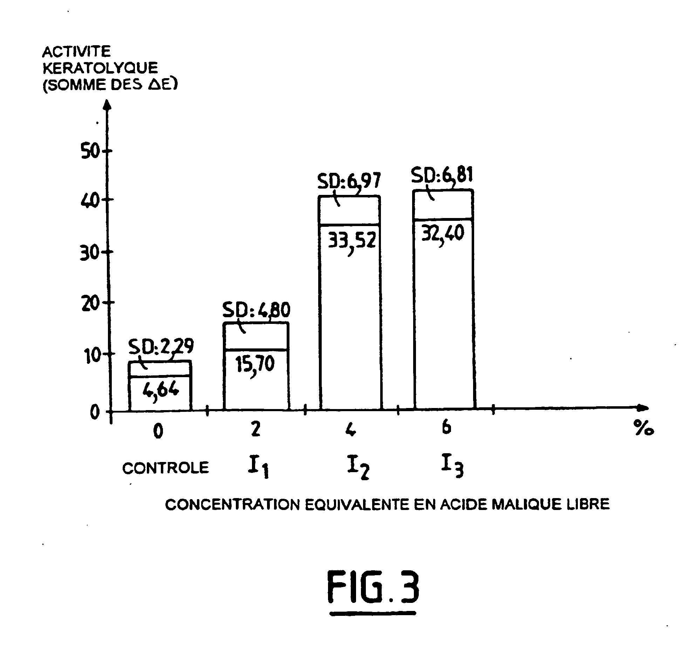

- la figure 1 montre des courbes standard qui peuvent être obtenues lors de la réalisation d'essais kératolytiques. Ces courbes sont obtenues en portant le temps en jour en abscisse et l'intensité de couleur telle que mesurée par un chromamètre, ici un Minolta®, en ordonnées. L'intensité de couleur au jour T0, référencée C0, est obtenue en appliquant une émulsion contenant la dihydroxyacétone (DHA) comme agent colorant comme décrit à l'exemple 20. La courbe I est obtenue en mesurant l'intensité de couleur avec le chromamètre en fonction du temps exprimé en jour, sans intervention particulière. La courbe II est obtenue en appliquant l'une des formulations kératolytiques testées, exprimées de la même manière. La différence entre les deux courbes, exprimée en tant que ΔE, mesure l'intensité kératolytique de la formulation kératolytique en fonction du temps. La somme des ΔE, qui représente l'aire ou surface définie entre les courbes I et II, représente la performance kératolytique globale de la formulation kératolytique testée et est l'objet de la figure 3 ;
- la figure 2 représente un exemple de diagramme baton obtenu avec une composition kératolytique comprenant un composé lipophile de l'exemple 1 de l'invention (I2) à base d'un mélange d'ester et d'anhydride d'acide malique/acide stéarique, correspondant à 4 % d'acide malique libre, en fonction du temps exprimé en jour en abscisse et également en fonction de l'intensité de couleur exprimée en différence de couleur ΔE en ordonnée, conformément à la figure 1 ; et
- la figure 3 représente les résultats d'essais obtenus avec divers composés de l'invention I1, I2 et I3 comme détaillé à l'exemple 19 en fonction de la concentration d'acide exprimée en acide libre équivalent et l'activité kératolytique en ordonnée exprimée sous forme d'aire ou surface résultant de la somme de toutes les différences de couleurs ΔE entre les courbes I et II tracées à la figure 1 et comme expliqué en relation avec la figure 1.

### Exemple 1 de l'invention

### Acide hydroxylé lipophile comprenant un mélange d'anhydride et d'ester d'acide malique et d'acide stéarique

100 g d'acide malique sont placés dans 500 ml d'eau déminéralisée. Le milieu réactionnel est ajusté à pH 11 par une solution d'hydroxyde de sodium (NaOH, 12N). Sous très forte agitation, type Ultraturrax ou Silverson (10 000 à 20 000 rpm), on ajoute lentement 100 g de chlorure d'acide stéarique.

Le pH passe en quelques dizaines de minutes d'une valeur de 11 à une valeur proche de 1, lorsqu'aucun tampon n'est ajouté au milieu réactionnel.

Après un temps de réaction de 1 h environ, l'ensemble est neutralisé jusqu'à un pH compris entre 2,0 et 7,0 par une solution d'hydroxyde de sodium (NaOH, 12N).

Il a été découvert selon la présente invention que le milieu réactionnel final peut être utilisé tel quel, sans purification ou séparation particulière pour constituer un principe actif cosmétique ou pharmaceutique ou dermatologique, et/ou comme agent émulsionnant ce qui constitue un avantage déterminant de la présente invention.

Des essais d'inocuité basés sur une absence d'irritation cutanée et occulaire ont été réalisés avec ce produit et font l'objet de l'exemple 18.

### Exemple 2 de l'invention

### Acide hydroxylé lipophile comprenant un mélange d'anhydride et d'ester d'acide glycolique et d'acide palmitique

On utilise le même procédé de greffage que celui décrit à l'exemple 1, mais on utilise l'acide glycolique en lieu et place de l'acide malique et le chlorure d'acide palmitique en lieu et place du chlorure d'acide stéarique.

On obtient ainsi un mélange d'anhydride et d'ester d'acide glycolique et d'acide palmitique, ainsi que les acides de départ qui n'ont pas réagi. Le milieu réactionnel brut final peut être utilisé tel quel.

### Exemple 3 de l'invention

### Acide hydroxylé lipophile comprenant un mélange d'anhydride et d'ester d'acide gluconique et d'acide laurique

On utilise le même procédé que celui décrit à l'exemple 1, si ce n'est que l'on utilise l'acide gluconique en lieu et place de l'acide malique et le chlorure de l'acide laurique en lieu et place du chlorure d'acide stéarique.

Le milieu réactionnel final peut être utilisé tel quel.

### Exemple 4 de l'invention

### Acide hydroxylé lipophile comprenant un mélange d'anhydride et d'ester d'acide salicylique et d'acide undécylénique

En procédant selon le procédé décrit à l'exemple 1, mais en utilisant l'acide salicylique en lieu et place de l'acide malique et le chlorure d'acide undécylénique en lieu et place du chlorure d'acide stéarique, on obtient le composé du titre en mélange avec les acides de départ n'ayant pas réagi.

Le milieu réactionnel final peut être utilisé tel quel.

### Exemple 5 de l'invention

### Acide hydroxylé lipophile comprenant un mélange d'anhydride et d'ester de polymères de divers acides et d'acide oléique et palmitique

### Exemple 5a

### Greffage d'un mélange de chlorure d'acide oléique et palmitique (20/80 p/p sur un polymère de l'acide glycolique

On procède comme décrit à l'exemple 1 mais on utilise un polymère de l'acide glycolique de poids moléculaire 10 000 à 700 000 D en lieu et place de l'acide malique, et un mélange de chlorure d'acide oléique et palmitique (20/80 p/p) en lieu et place du chlorure d'acide stéarique.

Le milieu réactionnel final peut être utilisé tel quel.

### Exemple 5b

### Produit de réaction d'un polymère de l'acide lactique avec un mélange de chlorure d'acide oléique et palmitique (20/80 p/p)

On procède comme décrit à l'exemple 1, si ce n'est qu'on utilise un polymère de l'acide lactique, poids moléculaire 10 000 à 700 000 D en lieu et place de l'acide malique, et un mélange du chlorure d'acide oléique et palmitique (20/80 p/p) en lieu et place du chlorure d'acide stéarique.

Le milieu réactionnel final peut être utilisé tel quel.

### Exemple 5c

### Produit de réaction d'un copolymère de l'acide glycolique et de l'acide lactique et d'un mélange de chlorure d'acide oléique et palmitique (20/80 p/p)

On procède comme décrit à l'exemple 1, si ce n'est qu'on utilise un copolymère de l'acide glycolique et de l'acide lactique de poids moléculaire 10 000 et 100 000 D, et un mélange du chlorure d'acide oléique et palmitique (20/80 p/p) en lieu et place du chlorure d'acide stéarique.

Le milieu réactionnel final peut être utilisé tel quel.

### Exemple 6 de l'invention

### Acide hydroxylé lipophile comprenant un mélange d'anhydride et d'ester de sérine et d'acide linolénique et stéarique

On procède comme décrit à l'exemple 1, si ce n'est que l'on utilise la sérine en lieu et place de l'acide malique, et un mélange de chlorures d'acide linolénique et stéarique (20/80 p/p) en lieu et place du chlorure d'acide stéarique.

Le milieu réactionnel final peut être utilisé tel quel.

### Exemple 7 de l'invention

### Acide hydroxylé lipophile comprenant un mélange d'anhydride et d'ester d'acide tartrique et d'acide acétique

On procède comme décrit à l'exemple 1, si ce n'est qu'on utilise l'acide tartrique en lieu et place de l'acide malique et de l'anhydride acétique en lieu et place du chlorure d'acide stéarique.

Le milieu réactionnel final peut être utilisé tel quel.

### Exemple 8 de l'invention

### Exemple 8a

### Acide hydroxylé lipophile comprenant un mélange d'anhydride et d'ester d'acide tartrique et d'acide succinique

On procède comme décrit à l'exemple 1 mais on utilise de l'acide tartrique en lieu et place de l'acide malique et l'anhydride succinique en lieu et place du chlorure d'acide stéarique.

Le milieu réactionnel final peut être utilisé tel quel.

### Exemple 8b

On procède comme décrit à l'exemple 1, si ce n'est que l'on utilise de l'acide tartrique en lieu et place de l'acide malique et l'anhydride maléique en lieu et place du chlorure d'acide stéarique.

Comme pour tous les autres exemples, le milieu réactionnel final peut être utilisé tel quel.

### Exemple 9 de l'invention

### Acide hydroxylé lipophile comprenant un mélange d'anhydride et d'ester d'acide lactique et d'acide stéarique

Alors que dans les exemples précédents 1 à 8, la réaction a lieu en solvant aqueux, il est décrit ci-après une réaction en solvant organique.

On place 1 mole d'acide lactique dans un mélange composé de 1000 ml de dichlorométhane et de 2 moles de triéthanolamine (TEA).

On ajoute ensuite 1 mole de chlorure d'acide stéarique goutte à goutte à ce mélange sous agitation.

Après quelques minutes de reaction, on élimine le solvant par distillation et le produit récupéré se présente sous la forme d'une poudre de couleur blanche.

Ce produit comprend d'une part l'acide hydroxylé lipophile recherché comprenant des fonctions anhydride et ester entre l'acide lactique et l'acide stéarique, ainsi que les acides de départ n'ayant pas réagi.

Ce produit formé par le milieu réactionnel final peut être utilisé tel quel.

### Exemple 10 de l'invention

### Acide hydroxylé lipophile comprenant les fonctions anhydride et ester d'acide lactique et d'acide stéarique

On procède comme décrit à l'exemple 9, si ce n'est qu'on utilise 2 moles de NaH, préalablement lavés à l'hexane, en lieu et place de 2 moles de TEA. La réaction s'effectue à une température proche de 0°C.

Ce produit formé par le milieu réactionnel final peut être utilisé tel quel.

### Exemple 11 de l'invention

### Acide hydroxylé lipophile comprenant des liaisons amides par réaction d'un acide hydroxylé avec une monoamine à radical alkyle en C10-C30

Cet exemple est général pour la préparation d'acide hydroxylé lipophile comprenant des liaisons amides.

En général, on placera 1 mole d'acide hydroxylé dans une quantité d'eau suffisante que l'on neutralise à pH environ égal à 7. On peut ajouter une quantité suffisante de tampon phosphate au milieu de manière à obtenir un tampon phosphate 0,5 M. On ajoute alors 1 mole d'un carbodiimide au mélange sous agitation.

L'ensemble peut être conservé sous agitation 1 h à température ambiante ou 24 h à 6°C.

On ajoute ensuite 1 mole d'une monoamine à radical alkyle en C10-C30 au mélange, sous agitation très puissante, du type Ultraturrax ou Sylverson. Après une réaction de 4 h à température ambiante, on ajuste l'ensemble à un pH compris entre 2 et 7, et de préférence entre 3 et 6, avec HCl,6N.

On peut ensuite réaliser une lyophilisation et éventuellement une stérilisation aux rayons béta ou gamma.

Le produit obtenu peut être utilisé tel quel comme principe actif d'une composition cosmétique ou pharmaceutique ou dermatologique.

On notera que la liaison covalente qui résulte de cette réaction fournit un amide hydroxylé et d'autres liaisons caractéristiques sont présentes entre l'amine et l'acide hydroxylé, comme les liaisons ioniques et les liaisons de type Van der Waals, qui participent efficacement à l'activité du produit.

### Exemple 12 de l'invention

### Acide hydroxylé lipophile comprenant des fonctions amides par réaction de l'acide malique avec une amine grasse

On procède comme décrit à l'exemple 11, si ce n'est que l'on utilise comme acide hydroxylé l'acide malique, comme amine une amine grasse constituée par la laurylamine ou la stéarylamine, et comme agent bifonctionnel de couplage un carbodiimide constitué par le chlorhydrate de N-(diméthylamino-3-propyl)-N'-éthylcarbodiimide.

Le milieu réactionnel peut être utilisé tel quel ou sous forme lyophilisée et éventuellement stérilisée aux rayons béta ou gamma. Ceci peut aussi être réalisé dans le cadre de tous les autres exemples.

### Exemple 13 de l'invention

### Acide hydroxylé lipophile comprenant des liaisons amides

### Exemple 13a

On procède comme décrit à l'exemple 12, mais on utilise comme tampon un tampon carbonate à une concentration 1 M. Le carbodiimide et l'amine sont placés ensemble dans le milieu réactionnel et le pH est ajusté à 7 tout au long de la réaction.

Le milieu réactionnel final peut être utilisé tel quel.

### Exemple 13b

On procède comme décrit à l'exemple 13a, puis on utilise un tampon borate à une concentration 1 M.

Le milieu réactionnel final peut être utilisé tel quel.

### Exemple 13c

On procède comme décrit à l'exemple 13a, mais on utilise un tampon citrate à une concentration de 0,5 M.

Le milieu réactionnel final peut être utilisé tel quel.

### Exemple 14 de l'invention

### Préparation d'acide hydroxylé lipophile comprenant des liaisons amides

On procède comme décrit à l'exemple 12, si ce n'est que l'on utilise au lieu d'un carbodiimide, comme agent bifonctionnel, le chlorure d'oxalyle (1 mole).

Le milieu réactionnel final peut être utilisé tel quel.

### Exemple 15 de l'invention

### Préparation d'acide hydroxylé lipophile par voie enzymatique

100 g de laurylamine sont placés à réagir avec 50 g d'acide malique et 25 g d'une lipase industrielle, par exemple la lipozyme® de Novo®. La réaction est conduite dans un réacteur clos sous agitation, à 60°C pendant 7 jours. Le rendement de greffage est de 10 % par rapport à la laurylamine de départ.

Le produit issu de ce greffage est utilisé tel quel dans la phase aqueuse d'une formulation cosmétique qui peut, par exemple, être réalisée à pH 3.

### Exemple 16 de l'invention

### Préparation d'acide hydroxylé lipophile comprenant des liaisons anhydrides et esters entre l'acide lactique et l'acide palmitique

On utilise le même procédé de greffage que celui décrit à l'exempel 1, mais on utilise l'acide lactique en lieu et place de l'acide malique, et le chlorure d'acide palmitique en lieu et place du chlorure d'acide stéarique.

On obtient ainsi un mélange d'anhydride et d'ester d'acide lactique, ainsi que les acides de départ qui n'ont pas réagi.

Le milieu réactionnel est avantageusement utilisé tel quel.

On observera que dans l'ensemble des exemples précédents, comme pour tous les produits de l'invention, ces produits sont utilisables comme principe actif d'une composition cosmétique, pharmaceutique et/ou dermatologique, ou comme agent émulsionnant, ou simultanément dans ces deux buts.

Des essais démontrant les activités des produits selon l'invention sont maintenant décrits'ci-après.

### Exemple 17 de l'invention

### Essais d'innocuité par absence d'irritation cutanée et oculaire

On réalise des essais d'innocuités à l'aide du produit lyophilisé et stérilisé par rayons gamma, sous forme d'une poudre pulvérulente très blanche, obtenue à l'exemple 1.

Les études d'irritation cutanée et oculaire sont réalisées selon des protocoles en accord avec les directives CEE N°404 (12 mai 1980), N°405 (24 février 1987), de la manière suivante :
a) Tout d'abord, le produit pur est utilisé sous forme pulvérulente et à pH7.
b) Le produit obtenu est utilisé à pH 5,5 sous forme d'une solution à 20 % en poids dans l'eau, ce qui représente 10 % en poids d'acide malique.
c) Le produit est utilisé à pH 3,5 sous forme d'une solution à 20 % en poids, ce qui représente 10 % en poids en acide malique.

Le réglage de ce pH pour chacun de ces produits est obtenu en variant l'étape finale de l'exemple 1. En effet, dans l'exemple 1 il est prévu qu'après un temps de réaction de 1 h environ, l'ensemble est neutralisé jusqu'à un pH compris entre 2 et 7 par une solution d'hydroxyde de sodium. On peut ainsi régler le pH du produit final.

Les tests réalisés selon les protocoles ci-dessus ont permis de constater que les produits sont apparus comme non irritants, ont été extrêmement bien tolérés et n'ont provoqué aucun signe d'irritation cutanée ou oculaire qu'ils soient au pH 7,5, 5 ou même 3,5.

Par ailleurs, une administration par voie orale de dose maximale de 5 g du produit de l'invention de l'exemple 1, par kilogramme de poids corporel, n'a provoqué aucune toxicité, ce test étant effectué en accord avec le protocole CEE concernant l'étude de la toxicité par voie orale de la directive CEE N°401 du 24 février 1987.

### Exemple 18 de l'invention

### Tests d'allergie in vivo sur des animaux

### Protocole expérimental

L'essai est réalisé selon le protocole expérimental décrit ci-après, adapté de la méthode décrite par Magnusson et Kligman (J. Invest. Derm. 1969, 52, 268-276), qui est reconnu par l'homme de l'art comme une méthode fiable et reproductible.

### 1 - Caractérisation du modèle expérimental

### 1 -1 -Animaux utlisés

- des cobayes albinos souche Hartley, provenant de chez Maury (24610 Villefranche de Lonchat, France),
- pesant environ 350 g au début de l'essai,
- identifiés par un marquage individuel à l'acide picrique.

Avant l'essai, les animaux sont maintenus pendant 6 jours dans les mêmes conditions que pendant l'essai.

### 1.2 - Stabulation

Les animaux sont maintenus dans 3 boîtes Makrolon de 46,5 cm x 31 cm x 19 cm dont le plancher est recouvert de sciures propres.

Un dispositif d'alimentation en nourriture du cobaye et une bouteille d'alimentation sont fixés au couvercle formant grille en acier inoxydable.

Les boîtes sont maintenues dans une pièce :
- éclairée d'une manière complémentaire avec de la lumière naturelle et artificielle,
- à l'air conditionné : le renouvellement de l'air a lieu 14 cycles par heure, la température est maintenue à 22°C± 1°C (limite inférieure et supérieure), l'humidité relative est maintenue à 52 % ± 8 (limite supérieure et inférieure).

### 1,3 - Nourriture

Les animaux reçoivent de l'eau du robinet et de la nourriture.

### 2 - Conditions d'essai

### 2.1 - Détermination de la concentration non irritante maximale par application topique

Cet essai est réalisé avec 3 cobayes qui ont reçu 3 semaines auparavant une injection d'adjuvant de Freund. Les concentrations différentes testées sous compresse semi-occlusive étaient de 1/1, 1/2, 1/4 et 1/8. Des dilutions ont été réalisées avec de l'eau distillée.

### 2.2 - Détermination de la concentration maximale irritante par injection intradermique

Ce contrôle n'était pas réalisé en raison du fait que la phase d'induction peut être réalisée avec du produit non dilué.

### 2.3 - Etude finale

Elle a été réalisée sur 30 animaux groupés par 15 :
GROUPE 1 (contrôle)
GROUPE 2 (traité).

### PHASE D'INDUCTION

Elle a été réalisée comme suit :
Groupe traité : Pour tous les animaux, au niveau de la zone scapulaire on réalise, sur les deux côtés de l'axe vertébral :
A D0 :
- Injection de 0,1 ml d'adjuvant de Freund à 50 % dans NaCl isotonique.
- Injection de 0,1 ml de produit non dilué.
- Injection de 0,1 ml d'un mélange de produit et d'adjuvant de Freund en volume égal.

A D7 :
Application sur la zone des injections de 1 ml de laurylsulfate de sodium à 10 % dans la vaseline.
A D8 :
Application pendant 48 heures sous compresse semi-occlusive de 0,5 ml du produit tel quel.

### Groupe de contrôle :

Les animaux de ce groupe recoivent le même traitement que ceux du groupe traité mais une solution saline est utilisée en lieu et place du produit.

Les animaux des deux groupes sont ensuite gardés pendant 2 semaines.

### PHASE DE CHALLENGE

Au jour D27, après qu'ils ont été soigneusement maintenus par des organes de maintien, les animaux des deux groupes reçoivent, sur une zone postérieure du dos, 0,5 ml du produit d'essai constitué par le produit d'invention obtenu à l'exemple 1, c'est-à-dire l'acide malique, à sa dose de concentration non irritante maximale (MNIC) et à sa demi-dose MNIC sous compresse semi-occlusive pendant 24 heures.

### 2.4 - Observations macroscopiques

On note l'érythème et/ou l'oedème 24 et 48 heures après enlèvement du tampon ou patch.

Les scores sont attribués selon l'échelle de notation suivante :

| | |
|---|---|
| Pas de réaction | 0 |
| Légère réaction | 1 |
| Réaction modérée | 2 |
| Réaction majeure | 3 |

L'érythème et l'oedème sont notés selon la même échelle de notation.

### 2.5 - Interprétation des résultats

Les scores obtenus pour l'érythème et l'oedème sont ajoutés.

Pour tous les animaux qui montrent un score égal ou supérieur à 2 après la phase de challenge, on considère qu'ils sont positifs.

Tous les autres signes (comme des picotements, des vésicules...) sont également pris en considération pour l'interprétation.

La puissance sensibilisante est définie par le pourcentage d'animaux positifs selon l'échelle suivante :

| Pourcentage | Classe | Classification |
|---|---|---|
| 0-8 | I | Très légère |
| 9-28 | II | Légère |
| 29-64 | III | Modérée |
| 65 - 80 | IV | Forte |
| 81 - 100 | V | Très forte |

### RESULTATS

### 1 - Concentrations à utiliser

Pour l'induction : 1/1
Pour la phase de challenge : 1/1 (MNIC) et 1/2 (1/2 MNIC)

### 2 - Détermination d'un pouvoir sensibilisant

Toute réaction microscopique a été enregistrée aussi bien dans le groupe de contrôle que dans le groupe traité quelle que soit la concentration utilisée.

### CONCLUSION

Dans les conditions expérimentales employées, le produit - acide malique lipophilisé selon l'invention utilisé à 20 % dans de l'eau distillée - de l'invention n'induisait pas de réaction macroscopiquee qui pouvait être liée à une sensibilisation chez le cobaye albino.

Le produit peut en conséquence être considéré comme étant hypoallergénique.

### Exemple 20 de l'invention

### Essais d'activité kératolytique in vivo sur des volontaires Caucasiens adultes

### A - Méthode

Afin de déterminer l'activité kératolytique des acides hydroxylés lipophilisés selon l'invention, des formulations cosmétiques contenant des quantités croissantes d'acides hydroxylés lipophilisés selon l'invention ont été réalisées, puis testés sur des panels de 10 volontaires de type caucasien adulte. Chaque panel a comparé une formule kératolytique à la formule placebo correspondante. Un site non traité a été préservé à titre de comparaison. Le test a été réalisé sur des zones circulaires de 25 mm de diamètre, délimitées sur la face interne des avant-bras à l'aide d'anneaux auto-adhésifs. Ces zones avaient été préalablement imprégnées à trois reprises et à 6 h d'intervalles, par une formulation cosmétique contenant 10 % de dihydroxyacétone.

Les crèmes testées ont été délivrées en quantité fixe de 1 ml au jour 1, 3, 5, 7, 10 et 12. Les régions concernées sont recouvertes pendant 1/2 heure avec une compresse aérée absorbante qui absorbe le produit annexé. Les différences en coloration sont notées visuellement avant chaque application.

### B - Compositions testées

Les compositions testées lors de cette étude sont les suivantes :
1 - Une composition placebo, réalisée à pH 5,5 ne contenant pas d'agent kératolytique, ayant la formulation centésimale suivante :
**Phase A**

| | |
|---|---|
| Dihydroxycétyl phosphate de sodium (et) isopropylhydroxycétyl éther | 2 % |
| Glycérine | 3 % |
| Propylène glycol | 2 % |
| Eau | qsp 100 % |

**Phase B**

| | |
|---|---|
| Glycol stéarate | 14% |
| Triisononanoine | 5% |
| Cocoate d'octyle | 6 % |

**Phase C**

| | |
|---|---|
| Agent bactéricide à base de butylèneglycol et de parabènes disponible dans le commerce sous la dénomination Bactéricide MB, société DRAGOCO | 1 % |
| Phénoxyéthanol et parabènes | 1 % |

Cette composition placebo est préparée de manière classique, en émulsionnant la phase B dans la phase A sous agitation, puis en ajoutant l'agent bactéricide de la phase C.
2 - Composition A₁ réalisée à pH 5.5 contenant 4 % d'acide malique
Pour la préparation de cette composition A₁, on procède comme décrit pour la fabrication de la composition placebo, mais la phase A contient 4 % de l'acide malique.
3 - Compositions de l'invention I₁,I₂,I₃
Ces compositions I₁, I₂, I₃ sont préparées comme décrit pour la composition placebo, si ce n'est que la phase A contient des quantités croissantes du composé selon l'invention de l'exemple 1 comprenant un mélange de liaison ester et anhydride d'acide malique/acide stéarique, la concentration équivalente de la partie acide malique dans le composé de l'invention représentant respectivement 2 %, 4 % et 6 % des compositions I₁, I₂, I₃. Ces deux compositions sont également préparées à pH 5,5
Pour régler le pH à 5,5 pour chacune de ces compositions placebo, A₁ et I₁, I₂ et I₃, on ajuste le pH à l'aide d'acide chlorhydrique concentré, ou de la soude concentrée.

### C - Résultats

Les applications répétées d'une formulation à base de dihydroxyacétone (DHA) permettent de colorer les couches supérieures de la peau, comme le ferait un produit "auto bronzant". La décoloration d'une zone ainsi traitée nécessite 15 jours. L'application, sur une telle zone, d'une formulation cosmétique contenant un actif kératolytique, va permettre d'obtenir une décoloration plus rapide. La mesure de cette décoloration tous les trois jours pendant 15 jours, permet d'étudier le pouvoir kératolytique des principes actifs étudiés.

Une telle décoloration est mesurée avec un chromamètre Minolta. Les paramètres de lumière L, a et b sont calculés sous forme d'une moyenne de 5 données expérimentales pour chaque sujet et pour chaque période surveillée.

La différence de couleur moyenne ΔE*ab = (ΔL²+Δa²+Δb²)^{1/2} est calculée entre chaque site traité et la zone de contrôle (colorée avec DHA mais non traitée avec l'une des compositions).

Si T₀ est le début de l'expérience et T_{F} la fin de l'expérience (voir figure 1), ΔE représente la variation en intensité de couleur entre la zone de contrôle définie par la courbe I, ici constituée par une ligne droite, s'étendant du point d'intensité de couleur C₀ à T₀ et du point d'intensité de couleur C_{F} à T_{F}, et une zone traitée avec une composition kératolytique définie par la ligne 2, figure 1, ici encore constituée par une ligne droite s'étendant du point d'intensité de couleur C₀ au point d'intensité de couleur C_{F} qui est ici obtenu au temps T_{ΔEmax}. Cette variation devrait augmenter jusqu'à un maximum (ΔEₘₐₓ) atteint pour T_{ΔEmax}, et ensuite devrait décroître.

De telles variations dépendent de la propriété kératolytique de chaque composition. La surface entre les deux courbes est directement proportionnelle à l'intensité de puissance kératolytique de chaque composition.

Ainsi, les applications des compositions indiquées ci-dessus, respectivement la composition placebo, la composition de comparaison A₁ et les compositions de l'invention I₁, I₂, I₃, selon la méthode décrite ci-dessus, rendent possible d'observer les résultats suivants :
a) La composition placebo fait disparaître une partie de la coloration, ce qui est notale dès le 8e jour. Cette remarque se confirme tout au long des 15 jours de l'expérience nécessaires pour l'obtention d'une décoloration complète. Cela signifie que cette préparation possède un pouvoir kératolytique faible mais présent. Ce pouvoir peut provenir du massage lors de l'application de la crème, ainsi que du pouvoir hydratant de la crème qui, en désolidarisant les coméocytes, permet ainsi une desquamation un peu plus rapide que la normale.
b) Les deux compositions kératolytiques respectivement A₁, I₁, I₂ et I₃ sont très fortement kératolytiques. En effet, 7 jours après le début des tests, la décoloration est déjà quasi-totale, ce qui signifie que le temps nécessaire pour provoquer la desquamation des couches colorées est divisé par deux, on passe de 15 jours à 7 jours. Ce résultat est confirmé par la mesure par chromamètre vis-à-vis du temps.
   Les mesures par chromamètre obtenues par rapport au temps pour chacune des compositions testées A₁, I₁, I₂ et I₃ sont rapportées au tableau I ci-dessous, exprimées comme différence ΔE d'intensité de couleur moyenne conformément au standard de la figure 1. 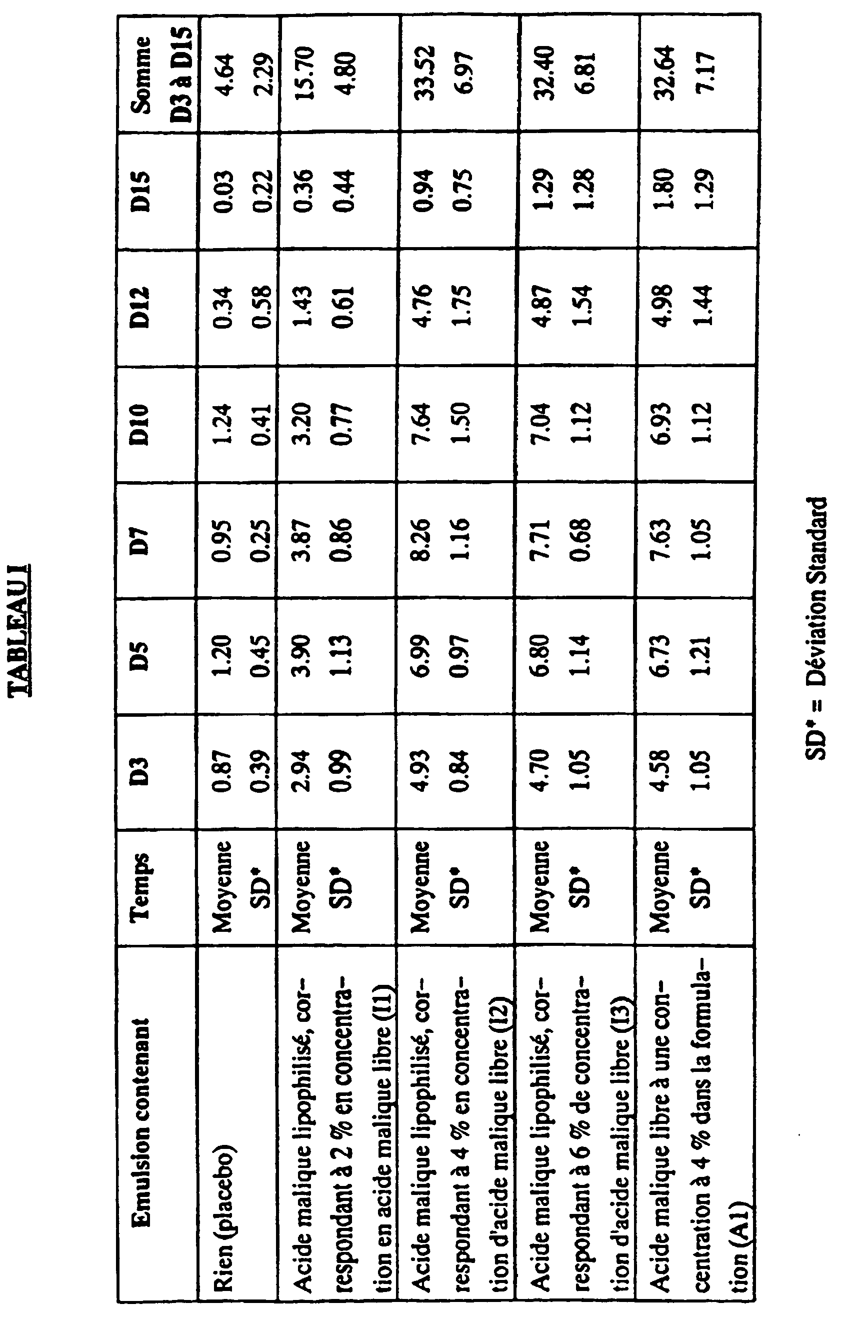
   A titre d'exemple, les différences d'intensité de couleur obtenue avec la composition de l'invention I2 est rapportée sous forme de diagramme bâton à la figure 2, les différences ΔE d'intensité de couleur étant représentées en noir et la déviation standard SD étant ajoutée sous forme d'un bâton gris.
   En outre, la somme de toutes les différences d'intensité de couleur ΔE obtenue avec la composition placébo, et les compositions de l'invention I1, I2 et I3 sont rapportées sur la figure 3 par rapport à la concentration en acide malique libre équivalente, en noir, la déviation standard SD étant ajoutée en gris.
   Il doit être noté que les résultats d'essais obtenus avec l'acide malique libre à 4% sous forme de contrôle positif mentionné au tableau I, ne sont pas montrés à la figure 3 puisque les bâtons obtenus sont essentiellement identiques à ceux de la composition de l'invention 12 et ne peuvent pas être différenciés de celle-ci.
   A partir des ces résultats d'essais, il peut être observé que, pour une même concentration équivalente en acide malique, la composition contenant de l'acide malique lipophilisé (I2) a un pouvoir ou intensité kératolytique aussi fort que la composition contenant l'acide malique libre (A1).
   L'acide malique lipophilisé selon l'invention et décrit à l'exemple 1 de la présente invention, a un pouvoir ou intensité kératolytique aussi fort que l'acide malique libre.
c) Parmi les deux compositions kératolytiques, respectivement A₁ et I₂, la composition I₂, réalisée avec l'acide malique lipophilisé selon la présente invention, a été parfaitement bien tolérée par les différents volontaires, et n'a provoquée aucun picotement, aucune irritation et aucune inflammation alors que des picotements et des rougeurs ont été perceptibles dans le cas de l'acide malique libre utilisé dans la formulation de la composition A₁.
d) Parmi toutes les compositions kératolytiques contenant l'acide malique lipophilisé, c'est-à-dire I1 (4,3 % d'acide malique lipophilisé, correspondant à une concentration équivalente en acide malique de 2 %), 12 (8,7 % d'acide malique lipophilisé, correspondant à une concentration équivalente en acide malique à 4 %) et 13 (13 % d'acide malique lipophilisé, correspondant à une concentration équivalent en acide malique de 6 %), 12 et 13 sont les compositions kératolytiques les plus puissantes (voir figure 3).

En augmentant, la concentration en acide malique lipophilisé dans les compositions, on obtient une augmentation en propriétés kératolytiques de telles compositions.

Une telle augmentation est stabilisée à un plateau pour des fortes concentrations, très probablement due à une capacité de stockage maximum de la peau.

On décrit maintenant ci-après divers exemples de formulation de composition cosmétique ou pharmaceutique et/ou dermatologique.

### Exemple 20 de l'invention

### Composition cosmétique à effet kératolytique

Cette Composition présente la composition centésimale suivante :
- Acide hydroxylé lipophile, acide malique/acide stéarique, de l'exemple 1 10 %
- Acide salicytique 0,5 %
- Urée 4 %
- Excipient cosmétique qsp 100 %

### Exemple 21 de l'invention

### Composition pharmaceutique effet anti-acnéique

Cette composition présente la composition centésimale suivante :
- acide hydroxyle lipophile, acide salicylique/acide undécytenique, de l'exemple 4 4 %
- Excipient pharmaceutique qsp 100 %

### Exemple 22 de l'invention

### Composition cosmétique à activité antipelliculaire

Cette composition présente les ingrédients suivants en pourcentage en poids :
- Acide hydroxylé lipophile de l'exemple 16 (acide malique/laury lamine) 10 %
- Excipient cosmétique qsp 100 %

### Exemple 23 de l'invention

### Composition Cosmétique hydratante

- Acide hydroxylé lipophile, de l'exemple 6 (serine/acide linolénique et steanque) 5%
- Urée 2 %
- Excipient cosmétique qsp 100 %

### Exemple 24 de l'invention

### Composition pharmaceutique pour traiter les peaux ichtyosiques, le psoriasis ou les eczémas

- Acide hydroxylé lipophile, de l'exemple 4 (acide salicylique/acide undécylénique) 6%
- Acide hydroxylé lipophile de l'exemple 2 (acide glycolique/acide palmitique) 6 %
- Excipient pharmaceutique qsp 100 %

### Exemple25 de l'invention

### Formulation anti-âge, restructurante

| Phases | | Ouantites(%) |
|---|---|---|
| A/ | Ether de polyéthylène glycol (2) et de stéaryl alcool | 3 |
| | Ether de polyéthylène glycol (21) et de stéaryl alcool | 2 |
| | Isostéaryl Isostéarate | 4 |
| | Huile de noyau d'abricot | 4 |
| | Huile de Carthame | 2 |
| | Diméthicone 556 | 2 |
| | Cétostéaryl Alcool | 3 |
| B/ | Glycérine | 5 |
| | Acide hydroxylé lipophile, de l'exemple 1 (acide malique/acide stéarique) | 6 |
| | Eau qsp | 100 |
| C/ | Phénoxyéthanol et mélange de parabènes | 0,5 |
| | Propylèneglycol | 0,5 |
| D/ | Phénoxyéthanol | 0,3 |
| | Alpha tocophérol | 0,05 |

Chauffer séparément, les phases A et B à 75°C, sous agitation modérée. Ajuster le pH de la phase B à la valeur de pH souhaitée. Verser A dans B sous agitation très violente (du type Silverson ou Uitratunax), puis laisser chuter la température sous agitation lente. A 30°C, rajouter les composants des phases C et D.

### Exemple 26 de l'invention

### Formulation anti-rides

| Phases | | Quantités (%) |
|---|---|---|
| A/ | Isostéaryl Isostéarate | 4 |
| | Huile de Carthame | 4 |
| | Oleyl Erucate | 2 |
| | Diméthicone | 5 |
| | Cétostéaryl Alcool | 3 |
| | Acide hydroxylé lipophile, de l'exemple 1 (acide malique/acide stéarique) | 3 |
| B/ | Glycérine | 5 |
| | Eau | qsp 100 |
| C/ | Phénoxyéthanol et mélange de paraben | 0,5 |
| | Propylène glycol | 0,5 |
| D/ | Phénoxyéthanol | 0,3 |

Chauffer séparément les phases A et B à 75°C, sous agitation modérée. Le pH de la formule est conditionné dans ce cas par le pH de l'acide hydroxylé lipophile. Verser A dans B sous agitation très violente (du type Silverson ou Ultraturrax), puis laisser chuter la température sous agitation lente. A 30°C, rajouter les composants des phases C et D. Si nécessaire, ajuster la préparation au pH désiré, à l'aide d'acide lactique par exemple.

A noter que le produit de l'invention est également utilisé en tant qu'agent émulsionnant dans cette formulation.

### Exemple 27 de l'invention

### Formulation peaux sèches, visage

| Phases | | Quantités (%) |
|---|---|---|
| A/ | Huile de bourrache | 2 |
| | Huile de Carthame | 4 |
| | Triglycéride Caprylique/Caprique | 6 |
| | Cétostéaryl Alcool | 3 |
| B/ | Glycérine | 5 |
| | Eau | qps 100 |
| | Acide hydroxylé lipophile, de l'exemple 2 (acide glycolique/acide palmitique) | 4 |
| C/ | Phénoxyéthanol et mélange de parabènes | 0,5 |
| | Propylèneglycol | 0,5 |
| D/ | Phénoxyéthanol | 0,3 |

Chauffer séparément les phases A et B à 75°C, sous agitation modérée. Le pH de la phase B est ajusté au pH de la formulation désiré. Verser A dans B sous agitation très violente (du type Silverson ou Ultraturrax), puis laisser chuter la température sous agitation lente. A 30°C, rajouter les composants des phases C et D. Si nécessaire, ajuster la préparation au pH désiré, à l'aide d'acide lactique par exemple.

A noter que le produit de l'invention est également utilisé en tant qu'agent émulsionnant dans la formulation (absence d'autres agents émulsionnants dans la formule).

## Revendications

1. Utilisation d'un acide hydroxylé lipophile comprenant au moins une fonction hydroxyle et au moins une fonction acide, **caractérisée en ce que** ledit acide hydroxylé, lipophile, comprend une chaîne hydrocarbonée hydrophobe ayant de 7 à 30 atomes de carbone greffée sur la fonction hydroxyle et/ou la fonction acide de l'acide hydroxylé lipophile par une liaison covalente choisie parmi le groupe consistant d'une liaison ester sur la fonction hydroxyle, d'une liaison anhydride sur la fonction acide et d'une liaison amide sur la fonction acide à la condition que dans le cas d'une liaison amide cette chaîne hydrocarbonée hydrophobe soit constituée par un radical alkyle ayant de 10 à 30 atomes de carbone résultant d'une monoamine, ou d'une combinaison de ces liaisons et ledit acide hydroxylé avant greffage de la chaîne hydrocarbonée hydrophobe est choisi parmi le groupe consistant d'un acide alpha-hydroxylé, choisi parmi l'acide glycolique, l'acide lactique, l'acide malique, l'acide tartrique, l'acide gluconique, l'acide méthyl lactique, l'acide mandélique, l'acide atrolactique, l'acide phényl lactique, l'acide glycérique, l'acide benzylique, l'acide alpha-hydroxyhexanoique, l'acide alpha-hydroxyheptanoique, l'acide alpha-hydroxyoctanoique ; ou un acide bêta-hydroxylé choisi parmi l'acide salicylique, la sérine ; un polymère ou un copolymère de cet acide hydroxyle, ou leurs mélanges ; comme agent cosmétique ou comme ingrédient actif;
pour la fabrication d'une composition cosmétique.

2. Utilisation d'un acide hydroxylé lipophile comprenant au moins une fonction hydroxyle et au moins une fonction acide, **caractérisée en ce que** ledit acide hydroxylé, lipophile, comprend une chaîne hydrocarbonée hydrophobe ayant de 7 à 30 atomes de carbone greffée sur la fonction hydroxyle et/ou la fonction acide de l'acide hydroxylé lipophile par une liaison covalente choisie parmi le groupe consistant d'une liaison ester sur la fonction hydroxyle, d'une liaison anhydride sur la fonction acide et d'une liaison amide sur la fonction acide à la condition que dans le cas d'une liaison amide cette chaîne hydrocarbonée hydrophobe soit constituée par un radical alkyle ayant de 10 à 30 atomes de carbone résultant d'une monoamine, ou d'une combinaison de ces liaisons et ledit acide hydroxylé avant greffage de la chaîne hydrocarbonée hydrophobe est choisi parmi le groupe consistant d'un acide alpha-hydroxylé, choisi parmi l'acide glycolique, l'acide lactique, l'acide malique, l'acide tartrique, l'acide gluconique, l'acide méthyl lactique, l'acide mandélique, l'acide atrolactique, l'acide phényl lactique, l'acide glycérique, l'acide benzylique, l'acide alpha-hydroxyhexanoique, l'acide alpha-hydroxyheptanoique, l'acide alpha-hydroxyoctanoique ; ou un acide bêta-hydroxylé, choisi parmi l'acide salicylique, la sérine ; un polymère ou un copolymère de cet acide hydroxylé, ou leurs mélanges ;
pour la fabrication d'une composition dermatologique et/ou pharmaceutique.

3. Utilisation d'un acide hydroxylé lipophile comprenant au moins une fonction hydroxyle et au moins une fonction acide, **caractérisée en ce que** ledit acide hydroxylé, lipophile, comprend une chaîne bydrocarbonée hydrophobe ayant de 7 à 30 atomes de carbone greffée sur la fonction hydroxyle et/ou la fonction acide de l'acide hydroxylé lipophile par une liaison covalente choisie parmi le groupe consistant d'une liaison ester sur la fonction hydroxyle, d'une liaison anhydride sur la fonction acide et d'une liaison amide sur la fonction acide à la condition que dans le cas d'une liaison amide cette chaîne hydrocarbonée hydrophobe soit constituée par un radical alkyle ayant de 10 à 30 atomes de carbone résultant d'une monoamine, ou d'une combinaison de ces liaisons et ledit acide hydroxylé avant greffage de la chaîne hydrocarbonée hydrophobe est choisi parmi le groupe consistant d'un acide alpha-hydroxylé, choisi parmi l'acide glycolique, l'acide lactique, l'acide malique, l'acide tartrique, l'acide gluconique, l'acide citrique, l'acide méthyl lactique, l'acide mandélique, l'acide atrolactique, l'acide phényl lactique, l'acide glycérique, l'acide benzylique, l'acide alpha-hydroxybutanoique, l'acide alpha-hydroxyhexanoique, l'acide alpha-hydroxyheptanoique, l'acide alpha-hydroxyoctanoique ; ou un acide bêta-hydroxylé, choisi parmi l'acide salicylique, la sérine ; un polymère ou un copolymère de cet acide hydroxylé, ou leurs mélanges ;
comme agent émulsionnant.

4. Utilisation selon l'une des revendications 1 à 3, **caractérisée en ce que** l'acide hydroxylé lipophile précité est le produit de réaction d'un acide hydroxylé choisi parmi le groupe consistant de l'acide malique, de l'acide glycolique, de l'acide gluconique, de l'acide salicylique, de l'acide lactique, de la sérine, d'un polymère de l'acide giycolique, d'un polymère de l'acide lactique, d'un copolymère de l'acide giycolique et de l'acide lactique ; et d'un composé comprenant la chaîne hydrocarbonée hydrophobe précitée choisi parmi le groupe consistant d'un halogénure ou anhydride d'acide stéarique, d'acide palmitique, d'acide myristique, d'acide laurique, d'acide undécylénique, d'acide undécanoïque, d'acide oléique, d'acide linoléique, d'acide linolénique, d'acide acétique, ou de leurs dérivés succiniques, ou maléiques.

5. Utilisation selon l'une des revendications 1 à 4, **caractérisée en ce que** le rapport en pourcentage entre l'acide hydroxylé et la chaîne hydrophobe est de 0,05 à 10, et de préférence de 0,05 à 2, en poids.

6. Utilisation selon l'une des revendications 1 à 5, **caractérisée en ce que** l'acide hydroxylé lipophile précité est sous une forme sèche, en particulier lyophilisée.

7. Utilisation selon l'une des revendications 1 à 6, **caractérisée en ce que** l'acide hydroxylé lipophile est présent dans le milieu réactionnel final avec les acides de départ n'ayant pas réagi.

8. Utilisation selon l'une quelconque des revendications précédentes, **caractérisée en ce que** l'acide gras hydroxylé lipophile est utilisé en tant qu'agent émulsionnant unique, ou en tant qu'agent co-émulsionnant.

9. Utilisation selon l'une des revendications précédentes, **caractérisée en ce qu'**il s'agit d'un acide hydroxylé lipophile comprenant un mélange d'anhydride et d'ester d'acide malique et d'acide stéarique.

10. Utilisation selon l'une des revendications précédentes, **caractérisée en ce que** l'acide hydroxylé lipophile comprend un mélange d'anhydride et d'ester d'acide glycolique et d'acide palmitique.

11. Utilisation selon l'une des revendications précédentes, **caractérisée en ce que** l'acide hydroxylé lipophile comprend un mélange d'anhydride et d'ester d'acide gluconique et d'acide laurique.

12. Utilisation selon l'une des revendications précédentes, **caractérisée en ce que** l'acide hydroxylé lipophile comprend un mélange d'anhydride et d'ester d'acide salicylique et d'acide undécylénique.

13. Utilisation selon l'une des revendications précédentes, **caractérisée en ce que** l'acide hydroxylé lipophile comprend un mélange d'anhydride et d'ester de polymères de l'acide glycolique de poids moléculaire 10000 à 700000 Dalton avec un mélange de chlorures d'acides oléique et palmitique de 20/80 p/p.

14. Utilisation selon l'une des revendications précédentes, **caractérisée en ce que** l'acide hydroxylé lipophile comprend un mélange d'anhydride et d'ester d'un polymère de l'acide lactique ayant un poids moléculaire de 10000 à 700000 Dalton avec un mélange de chlorures d'acides oléique et palmitique de 20/80 p/p,

15. Utilisation selon l'une des revendications précédentes, **caractérisée en ce que** l'acide hydroxylé lipophile comprend un mélange d'anhydride et d'ester résultant du produit de réaction d'un copolymère de l'acide glycolique et de l'acide lactique ayant un poids moléculaire de 10000 à 100000 Dalton avec un mélange de chlorures d'acides oléique et palmitique de 20/80 p/p.

16. Utilisation selon l'une des revendications précédentes, **caractérisée en ce que** l'acide hydroxylé lipophile comprend un mélange d'anhydride et d'ester de sérine et d'acide linoléique et stéarique de 20/80 p/p.

17. Utilisation selon l'une quelconque des revendications précédentes, **caractérisée en ce que** l'acide hydroxylé lipophile comprend un mélange d'anhydride et d'ester d'acide tartrique et d'acide acétique.

18. Utilisation selon l'une quelconque des revendications précédentes, **caractérisée en ce que** l'acide hydroxylé lipophile comprend un mélange d'anhydride et d'ester d'acide tartrique et d'acide succinique.

19. Utilisation selon l'une quelconque des revendications précédentes, **caractérisée en ce que** l'acide hydroxylé lipophile comprend un mélange d'anhydride et d'ester d'acide tartrique et d'acide maléique.

20. Utilisation selon l'une des revendications précédentes, **caractérisée en ce que** l'acide hydroxylé lipophile comprend un mélange d'anhydride et d'ester d'acide lactique et d'acide stéarique.

21. Utilisation selon l'une des revendications précédentes, **caractérisée en ce que** l'acide hydroxylé lipophile comprend des liaisons amides par réaction de l'acide malique avec une amine grasse constituée par la laurylamine ou la stéarylamine.

22. Utilisation selon l'une des revendications précédentes, **caractérisée en ce que** l'acide hydroxylé lipophile comprend des fonctions amides par réaction de l'acide malique avec la laurylamine.

23. Utilisation selon l'une des revendications précédentes, **caractérisée en ce que** l'acide hydroxylé lipophile comprend des liaisons anhydride et ester entre l'acide lactique et l'acide palmitique.

24. Utilisation selon une quelconque des revendications 1, 4 à 8, **caractérisée en ce qu'**il s'agit de l'acide malique lipophile utilisé pour une activité choisie parmi une activité kératolytique, une activité antipelliculaire, une activité anti-âge et une activité anti-rides.

25. Utilisation selon l'une des revendications 2, 4 à 8, **caractérisée en ce qu'**il s'agit de l'acide undécylénique lipophile utilisé pour une activité anti-acnéique.

26. Utilisation selon l'une des revendications 2. 4 à 8, **caractérisée en ce qu'**il s'agit de l'acide salicylique lipophile utilisé pour une activité anti-acnéique.

27. Utilisation selon l'une des revendications 1, 4 à 8, **caractérisée en ce qu'**il s'agit de l'acide glycolique lipophile utilisé pour réaliser une activité hydratante permettant le traitement des peaux sèches.

28. Utilisation selon l'une quelconque des revendications 1, 4 à 8, **caractérisée en ce qu'**il s'agit de la sérine lipophile utilisée pour une activité hydratante.

29. Utilisation selon l'une des revendications 2, 4 à 8, **caractérisée en ce qu'**il s'agit de l'acide salicylique et/ou glycolique lipophile utilisé pour traiter les peaux ichtyosiques, psoriasis ou les eczémas.

30. Acide hydroxylé lipophile comprenant au moins une fonction hydroxyle et au moins une fonction acide, **caractérisé en ce que** ledit acide hydroxylé, lipophile, comprend une chaîne hydrocarbonée hydrophobe ayant de 7 à 30 atomes de carbone greffée sur la fonction hydroxyle et/ou la fonction acide de l'acide hydroxylé lipophile par une liaison covalente choisie parmi le groupe consistant d'une liaison ester sur la fonction hydroxyle, d'une liaison anhydride sur la fonction acide et d'une liaison amide sur la fonction acide à la condition que dans le cas d'une liaison amide cette chaîne hydrocarbonée hydrophobe soit constituée par un radical alkyle ayant de 10 à 30 atomes de carbone résultant d'une monoamine, ou d'une combinaison de ces liaisons et ledit acide hydroxylé avant greffage de la chaîne hydrocarbonée hydrophobe est choisi parmi le groupe consistant d'un acide alpha-hydroxylé, choisi parmi l'acide glycolique, l'acide lactique, l'acide malique, l'acide tartrique, l'acide gluconique, l'acide méthyl lactique, l'acide mandélique, l'acide atrolactique, l'acide phényl lactique, l'acide glycérique, l'acide benzylique, l'acide alpha-hydroxyhexanoique, l'acide alpha-hydroxyheptanoique, l'acide alpha-hydroxyoctanoique ; ou un acide bêta-hydroxylé, choisi parmi l'acide salicylique, la sérine ; un polymère ou un copolymère de cet acide hydroxylé, ou leurs mélanges.

31. Acide hydroxylé lipophile selon la revendication 30 **caractérisé en ce que** l'acide hydroxylé hydrophile précité est le produit de réaction d'un acide hydroxylé choisi parmi le groupe consistant de l'acide malique, de l'acide glycolique, de l'acide gluconique, de l'acide salicylique, de l'acide lactique, de la sérine, d'un polymère de l'acide glycolique, d'un polymère de l'acide lactique, d'un copolymère de l'acide glycolique et de l'acide lactique ; et d'un composé comprenant la chaîne hydrocarbonée hydrophobe précitée choisi parmi le groupe consistant d'un halogénure ou anhydride d'acide stéarique, d'acide palmitique, d'acide myristique, d'acide laurique, d'acide undécylénique, d'acide undécanoïque, d'acide oléique, d'acide linoléique, d'acide linolénique, d'acide acétique, ou de leurs dérivés succiniques, ou maléiques.

32. Acide hydroxylé lipophile selon la revendication 30 ou 31, **caractérisé en ce que** le rapport en pourcentage entre l'acide hydroxylé et la chaîne hydrophobe 0,05 est de 0,05 à 10 et de préférence de 0,05 à 2, en poids.

33. Acide hydroxyié lipophile selon l'une des revendications 30 à 32, **caractérisé en ce que** l'acide hydroxylé lipophile précité est sous une forme sèche, en particulier lyophilisée.

34. Acide hydroxylé lipophile selon l'une des revendications 30 à 33, **caractérisé en ce que** l'acide bydroxylé lipophile est présent dans le milieu réactionnel final avec les acides de départ n'ayant pas réagi.

35. Acide hydroxylé lipophile selon l'une quelconque des revendications 30 à 34, **caractérisé en ce que** l'acide gras hydroxylé lipophile est utilisé en tant qu'agent émulsionnant unique, ou en tant qu'agent co-émulsionnant.

36. Acide hydroxylé lipophile selon l'une des revendications 30 à 35, **caractérisé en ce qu'**il s'agit d'un acide hydroxylé lipophile comprenant un mélange d'anhydride et d'ester d'acide malique et d'acide stéarique.

37. Acide hydroxylé lipophile selon l'une des revendications 30 à 36, **caractérisé en ce que** l'acide hydroxylé lipophile comprend un mélange d'anhydride et d'ester d'acide glycolique et d'acide palmitique.

38. Acide hydroxylé lipophile selon l'une des revendications 30 à 37, **caractérisé en ce que** l'acide hydroxylé lipophile comprend un mélange d'anhydride et d'ester d'acide gluconique et d'acide laurique.

39. Acide hydroxylé lipophile selon l'une des revendications 30 à 38, **caractérisé en ce que** l'acide hydroxylé lipophile comprend un mélange d'anhydride et d'ester d'acide salicylique et d'acide undécylénique.

40. Acide hydroxylé lipophile selon l'une des revendications 30 à 39,
**caractérisé en ce que** l'acide hydroxylé lipophile comprend un mélange d'anhydride et d'ester de polymères de l'acide glycolique de poids moléculaire 10 000 à 700 000 Dalton avec un mélange de chlorures d'acides oléique et palmitique de 20/80 p/p.

41. Acide hydroxylé lipophile selon l'une des revendications 30 à 40, **caractérisé en ce que** l'acide hydroxylé lipophile comprend un mélange d'anhydride et d'ester d'un polymère de l'acide lactique ayant un poids moléculaire de 10 000 à 700 000 Dalton avec un mélange de chlorures d'acides oléique et palmitique de 20/80 p/p.

42. Acide hydroxylé lipophile selon l'une des revendications 30 à 41, **caractérisé en ce que** l'acide hydroxylé lipophile comprend un mélange d'anhydride et d'ester résultant du produit de réaction d'un copolymère de l'acide glycolique et de l'acide lactique ayant un poids moléculaire de 10 000 à 100 000 Dalton avec un mélange de chlorures d'acides oléique et palmitique de 20/80 p/p.

43. Acide hydroxylé lipophile selon l'une des revendications 30 à 42, **caractérisé en ce que** l'acide hydroxylé lipophile comprend un mélange d'anhydride et d'ester de sérine et d'acide linolénique et stéarique de 20/80 p/p.

44. Acide hydroxylé lipophile selon l'une quelconque des revendications 30 à 43, **caractérisé en ce que** l'acide hydroxylé lipophile comprend un mélange d'anhydride et d'ester d'acide tartrique et d'acide acétique.

45. Acide hydroxylé lipophile selon l'une quelconque des revendications 30 à 44 **caractérisé en ce que** l'acide hydroxylé lipophile comprend un mélange d'anhydride et d'ester d'acide tartrique et d'acide succinique.

46. Acide hydroxylé lipophile selon l'une quelconque des revendications 30 à 45. **caractérisé en ce que** l'acide hydroxylé lipophile comprend un mélange d'anhydride et d'ester d'acide tartrique et d'acide maléique.

47. Acide hydroxylé lipophile selon l'une des revendications 30 à 46, **caractérisé en ce que** l'acide hydroxylé lipophile comprend un mélange d'anhydride et d'ester d'acide lactique et d'acide stéarique.

48. Acide hydroxylé lipophile selon l'une des revendications 30 à 36. **caractérisé en ce que** l'acide hydroxylé lipophile comprend des liaisons amides par réaction de l'acide malique avec une amide grasse constituée par la laurylamine ou la stéarylamine.

49. Acide hydroxylé lipophile selon la revendication 48, **caractérisé en ce que** l'acide hydroxylé lipophile comprend des fonctions amides par réaction de l'acide malique avec la laurylamine.

50. Acide hydroxylé lipophile selon l'une des revendications 30 à 49, **caractérisé en ce que** l'acide hydroxylé lipophile comprend des liaisons anhydride et ester entre l'acide lactique et l'acide palmitique.

51. Composition cosmétique, **caractérisée en ce qu'**elle comprend comme principe actif un acide hydroxylé lipophile, tel que défini à l'une quelconque des revendications 30 à 50.

52. Composition pharmaceutique et/ou dermatologique, **caractérisée en ce qu'**elle comprend un acide hydroxylé lipophile, tel que défini à l'une quelconque des revendications 30 à 50.

53. Composition selon la revendication 51 ou 52, **caractérisée en ce qu'**elle comprend l'acide hydroxylé lipophile précité simultanément comme principe actif et comme agent émulsionnant.

54. Composition selon l'une des revendications 51 à 53, **caractérisée en ce que** la proportion d'acide hydroxylé lipophile est comprise entre 0,1 % et 50 % en poids de la composition finale, de préférence entre 1 % et 20 %, en poids de la composition finale.

55. Composition comprenant comme agent émulsionnant au moins un acide hydroxylé lipophile, tel que défini à l'une quelconque des revendications 30 à 50, en particulier à une concentration comprise entre 0,1 et 50 % en poids de la composition finale, de préférence entre 1 et 20 % en poids de la composition finale.

56. Composition selon la revendication 55, **caractérisée en ce que** l'acide hydroxylé lipophile précité est utilisé en tant qu'agent émulsionnant unique ou en tant qu'agent co-émulsionnant.

57. Méthode cosmétique de traitement kératolytique de zone(s) de la peau en ayant besoin, comprenant l'application auxdites zones de la peau d'une quantité efficace kératolytique d'au moins un acide hydroxylé lipophile, tel que défini à l'une quelconque des revendications 30 à 50.

58. Méthode cosmétique pour réaliser une exfoliation chimique de la peau, pour stimuler les cellules de la peau, pour améliorer l'élasticité et la cohésion de la peau, pour dépigmenter la peau, pour hydrater la peau, pour réaliser un effet anti-rides de la peau, un effet anti-vieillissement sur la peau, un effet anti-pelliculaire sur des zones choisies des cheveux et du cuir chevelu, **caractérisée en ce qu'**elle comprend l'application sur la peau, la chevelure, le cuir chevelu en ayant besoin d'une quantité efficace d'un acide hydroxylé lipophile, tel que défini à l'une quelconque des revendications 30 à 50.

## Patentansprüche

1. Verwendung einer lipophilen hydroxylierten Säure, die wenigstens eine Hydroxylfunktion umfasst und wenigstens eine Säurefunktion, **dadurch gekennzeichnet, dass** die hydroxylierte lipophile Säure eine hydrophobe Kohlenwasserstoffkette mit 7 bis 30 Kohlenstoffatomen umfasst, die gepfropft ist an der Hydroxylfunktion und/oder der Säurefunktion der hydroxylierten lipophilen Säure durch eine kovalente Bindung, die gewählt ist aus der Gruppe, die besteht aus einer Esterbindung an der Hydroxylfunktion, einer Anhydridbindung an der Säurefunktion und einer Amidbindung an der Säurefunktion mit der Bedingung, dass in dem Fall einer Amidbindung diese hydrophobe Kohlenwasserstoffkette entweder aus einem Alkylrest mit 10 bis 30 Kohlenstoffatomen besteht, der aus einem Monoamin resultiert, oder einer Kombination dieser Bindungen, und die hydroxylierte Säure vor Pfropfen der hydrophoben Kohlenwasserstoffkette, gewählt ist aus der Gruppe, die besteht aus einer alpha-hydroxylierten Säure, gewählt unter Glykolsäure, Milchsäure, Maleinsäure, Weinsäure, Gluconsäure, Methylmilchsäure, Mandelsäure, Atrolactinsäure, Phenylmilchsäure, Glycerinsäure, Benzylsäure, alpha-Hydroxyhexansäure, alpha-Hydroxyheptansäure, alpha-Hydroxyoctansäure oder einer beta-hydroxylierten Säure, gewählt unter Salicylsäure, Serin; einem Polymer oder Co-Polymer dieser hydroxylierten Säure oder deren Gemischen; als kosmetisches Mittel oder als Wirkstoff;
zur Herstellung einer kosmetischen Zusammensetzung.

2. Verwendung einer lipophilen hydroxylierten Säure, die wenigstens eine Hydroxylfunktion umfasst und wenigstens eine Säurefunktion, **dadurch gekennzeichnet, dass** die hydroxylierte lipophile Säure eine hydrophobe Kohlenwasserstoffkette mit 7 bis 30 Kohlenstoffatomen umfasst, die gepfropft ist an der Hydroxylfunktion und/oder der Säurefunktion der hydroxylierten lipophilen Säure durch eine kovalente Bindung, die gewählt ist aus der Gruppe, die besteht aus einer Esterbindung an der Hydroxylfunktion, einer Anhydridbindung an der Säurefunktion und einer Amidbindung an der Säurefunktion mit der Bedingung, dass in dem Fall einer Amidbindung diese hydrophobe Kohlenwasserstoffkette entweder aus einem Alkylrest mit 10 bis 30 Kohlenstoffatomen besteht, der aus einem Monoamin resultiert, oder einer Kombination dieser Bindungen, und die hydroxylierte Säure vor Pfropfen der hydrophoben Kohlenwasserstoffkette, gewählt ist aus der Gruppe, die besteht aus einer alpha-hydroxylierten Säure, gewählt unter Glycolsäure, Milchsäure, Maleinsäure, Weinsäure, Gluconsäure, Methylmilchsäure, Mandelsäure, Atrolactinsäure, Phenylmilchsäure, Glycerinsäure, Benzylsäure, alpha-Hydroxyhexansäure, alpha-Hydroxyheptansäure, alpha-Hydroxyoctansäure oder einer beta-hydroxylierten Säure, gewählt unter Salicylsäure, Serin; einem Polymer oder Co-Polymer dieser hydroxylierten Säure oder deren Gemischen;
zur Herstellung einer dermatologischen und/oder pharmazeutischen Zusammensetzung.

3. Verwendung einer lipophilen hydroxylierten Säure, die wenigstens eine Hydroxylfunktion umfasst und wenigstens eine Säurefunktion, **dadurch gekennzeichnet, dass** die hydroxylierte lipophile Säure eine hydrophobe Kohlenwasserstoffkette mit 7 bis 30 Kohlenstoffatomen umfasst, die gepfropft ist an der Hydroxylfunktion und/oder der Säurefunktion der hydroxylierten lipophilen Säure durch eine kovalente Bindung, die gewählt ist aus der Gruppe, die besteht aus einer Esterbindung an der Hydroxylfunktion, einer Anhydridbindung an der Säurefunktion und einer Amidbindung an der Säurefunktion mit der Bedingung, dass in dem Fall einer Amidbindung diese hydrophobe Kohlenwasserstoffkette entweder aus einem Alkylrest mit 10 bis 30 Kohlenstoffatomen besteht, der aus einem Monoamin resultiert, oder einer Kombination dieser Bindungen, und die hydroxylierte Säure vor Pfropfen der hydrophoben Kohlenwasserstoffkette, gewählt ist aus der Gruppe, die besteht aus einer alpha-hydroxylierten Säure, gewählt unter Glycolsäure, Milchsäure, Maleinsäure, Weinsäure, Gluconsäure, Methylmilchsäure, Mandelsäure, Atrolactinsäure, Phenylmilchsäure, Glycerinsäure, Benzylsäure, alpha-Hydroxybutansäure, alpha-Hydroxyhexansäure, alpha-Hydroxyheptansäure, alpha-Hydroxyoctansäure oder einer beta-hydroxylierten Säure, gewählt unter Salicylsäure, Serin; einem Polymer oder Co-Polymer dieser hydroxylierten Säure oder deren Gemischen;
als Emulsionsmittel.

4. Verwendung nach einem der Ansprüche 1 bis 3, **dadurch gekennzeichnet, dass** die hydroxylierte oben genannte lipophile Säure das Produkt einer Reaktion einer hydroxylierten Säure ist, die gewählt ist aus der Gruppe, die besteht aus Maleinsäure, Glycolsäure, Glyconsäure, Salicylsäure, Milchsäure, Serin, von einem Polymer von Glycolsäure, von einem Polymer von Milchsäure, von einem Co-Polymer von Glycolsäure und Milchsäure; und einer Verbindung, die eine hydrophobe oben genannte Kohlenwasserstoffkette umfasst, gewählt aus der Gruppe, die besteht aus einem Halogenid oder Anhydrid von Stearinsäure, Palmitinsäure, Myristinsäure, Laurinsäure, Undecylensäure, Undecansäure, Ölsäure, Linolsäure, Linolensäure, Essigsäure oder deren Succin- oder Maleinderivaten.

5. Verwendung nach einem der Ansprüche 1 bis 4, **dadurch gekennzeichnet, dass** das prozentuale Verhältnis zwischen der hydroxylierten Säure und der hydrophoben Kette 0,05 bis 10 und vorzugsweise 0,05 bis 2 Gew.-% ist.

6. Verwendung nach einem der Ansprüche 1 bis 5, **dadurch gekennzeichnet, dass** die hydroxylierte lipophile oben genannte Säure in trockener Form, insbesondere lyophilisiert vorliegt.

7. Verwendung nach einem der Ansprüche 1 bis 6, **dadurch gekennzeichnet, dass** die lipophile hydroxylierte Säure in dem endgültigen Reaktionsmedium mit Ausgangssäuren vorliegt, die nicht reagiert haben.

8. Verwendung nach einem der vorhergehenden Ansprüche, **dadurch gekennzeichnet, dass** die lipophile hydroxylierte Fettsäure als einziges Emulsionsmittel verwendet wird oder als Co-Emulsionsmittel.

9. Verwendung nach einem der vorhergehenden Ansprüche, **dadurch gekennzeichnet, dass** es sich um eine lipophile hydroxylierte Säure handelt, die ein Gemisch von Anhydrid und Ester von Maleinsäure und Stearinsäure umfasst.

10. Verwendung nach einem der vorhergehenden Ansprüche, **dadurch gekennzeichnet, dass** die lipophile hydroxylierte Säure ein Gemisch von Anhydrid und Ester von Glykolsäure und Palmitinsäure umfasst.

11. Verwendung nach einem der vorhergehenden Ansprüche, **dadurch gekennzeichnet, dass** die lipophile hydroxylierte Säure ein Gemisch von Anhydrid und Ester von Gluconsäure und Laurinsäure umfasst.

12. Verwendung nach einem der vorhergehenden Ansprüche, **dadurch gekennzeichnet, dass** die lipophile hydroxylierte Säure ein Gemisch von Anhydrid und Ester von Salicylsäure und Undecylensäure umfasst.

13. Verwendung nach einem der vorhergehenden Ansprüche, **dadurch gekennzeichnet, dass** die lipophile hydroxylierte Säure ein Gemisch von Anhydrid und Ester von Polymeren von Glycolsäure mit einem Molekulargewicht von 10.000 bis 700.000 Dalton mit einem Gemisch von Chloriden von Öl- und Palmitinsäuren zu 20/80 w/w umfasst.

14. Verwendung nach einem der vorhergehenden Ansprüche, **dadurch gekennzeichnet, dass** die lipophile hydroxylierte Säure ein Gemisch von Anhydrid und Ester von einem Polymer von Milchsäure mit einem Molekulargewicht von 10.000 bis 700.000 Dalton mit einem Gemisch von Chloriden von Öl- und Palmitinsäuren zu 20/80 w/w umfasst.

15. Verwendung nach einem der vorhergehenden Ansprüche, **dadurch gekennzeichnet, dass** die lipophile hydroxylierte Säure ein Gemisch von Anhydrid und Ester umfasst, resultierend aus dem Reaktionsprodukt eines Co-Polymers von Glycolsäure und Milchsäure mit einem Molekulargewicht von 10.000 bis 700.000 Dalton mit einem Gemisch von Chloriden von ÖI- und Palmitinsäuren zu 20/80 w/w.

16. Verwendung nach einem der vorhergehenden Ansprüche, **dadurch gekennzeichnet, dass** die lipophile hydroxylierte Säure ein Gemisch von Anhydrid und Ester von Serin und Linol- und Stearinsäure zu 20/80 w/w umfasst.

17. Verwendung nach einem der vorhergehenden Ansprüche, **dadurch gekennzeichnet, dass** die lipophile hydroxylierte Säure ein Gemisch von Anhydrid und von Ester von Weinsäure und Essigsäure umfasst.

18. Verwendung nach einem der vorhergehenden Ansprüche, **dadurch gekennzeichnet, dass** die hydroxylierte lipophile Säure ein Gemisch von Anhydrid und Ester von Weinsäure und Bernsteinsäure umfasst.

19. Verwendung nach einem der vorhergehenden Ansprüche, **dadurch gekennzeichnet, dass** die lipophile hydroxylierte Säure ein Gemisch von Anhydrid und Ester von Weinsäure und Maleinsäure umfasst.

20. Verwendung nach einem der vorhergehenden Ansprüche, **dadurch gekennzeichnet, dass** die lipophile hydroxylierte Säure ein Gemisch von Anhydrid und Ester von Milchsäure und Stearinsäure umfasst.

21. Verwendung nach einem der vorhergehenden Ansprüche, **dadurch gekennzeichnet, dass** die hydroxylierte lipophile Säure Amidbindungen durch Reaktion von Maleinsäure mit einem Fettamin umfasst, das durch Laurylamin oder Stearylamin gebildet wird.

22. Verwendung nach einem der vorhergehenden Ansprüche, **dadurch gekennzeichnet, dass** die hydroxylierte lipophile Säure Amidfunktionen durch Reaktion der Maleinsäure mit Laurylamin umfasst.

23. Verwendung nach einem der vorhergehenden Ansprüche, **dadurch gekennzeichnet, dass** die lipophile hydroxylierte Säure Anhydridbindungen und Esterbindungen unter Milchsäure und Palmitinsäure umfasst.

24. Verwendung nach einem der Ansprüche 1, 4 bis 8, **dadurch gekennzeichnet, dass** es sich um lipophile Maleinsäure handelt, die für eine Wirkung verwendet wird, die gewählt ist unter keratinolytischer Wirkung, Anti-Schuppen-Wirkung, AntiAlterungs-Wirkung und einer Anti-Falten- Wirkung.

25. Verwendung nach einem der Ansprüche 2, 4 bis 8, **dadurch gekennzeichnet, dass** es sich um Undecylensäure handelt, die lipophil ist und verwendet wird für eine Anti-Akne-Wirkung.

26. Verwendung nach einem der Ansprüche 2, 4 bis 8, **dadurch gekennzeichnet, dass** es sich um lipophile Salicylsäure handelt, die für eine Anti-Akne-Wirkung verwendet wird.

27. Verwendung nach einem der Ansprüche 1, 4 bis 8, **dadurch gekennzeichnet, dass** es sich um lipophile Glykolsäure handelt, die verwendet wird, um eine befeuchtende Wirkung zu verwirklichen, die die Behandlung der trockenen Haut erlaubt.

28. Verwendung nach einem der Ansprüche 1, 4 bis 8, **dadurch gekennzeichnet, dass** es sich um lipophiles Serin handelt, das für eine befeuchtende Wirkung verwendet wird.

29. Verwendung nach einem der Ansprüche 2, 4 bis 8, **dadurch gekennzeichnet, dass** es sich um Salicylsäure und/oder Glykolsäure handelt, die lipophil ist und verwendet wird, um die Ichthyose-, Psoriasis-Haut oder Ekzeme zu behandeln.

30. Lipophile hydroxylierte Säure, die wenigstens eine Hydroxylfunktion umfasst und wenigstens eine Säurefunktion, **dadurch gekennzeichnet, dass** die hydroxylierte lipophile Säure eine hydrophobe Kohlenwasserstoffkette mit 7 bis 30 Kohlenstoffatomen umfasst, die gepfropft ist an der Hydroxylfunktion und/oder der Säurefunktion der hydroxylierten lipophilen Säure durch eine kovalente Bindung, die gewählt ist aus der Gruppe, die besteht aus einer Esterbindung an der Hydroxylfunktion, einer Anhydridbindung an der Säurefunktion und einer Amidbindung an der Säurefunktion mit der Bedingung, dass in dem Fall einer Amidbindung diese hydrophobe Kohlenwasserstoffkette entweder aus einem Alkylrest mit 10 bis 30 Kohlenstoffatomen besteht, resultierend aus einem Monoamin, oder einer Kombination dieser Bindungen, und die hydroxylierte Säure vor Pfropfen der hydrophoben Kohlenwasserstoffkette, gewählt ist aus der Gruppe, die besteht aus einer alpha-hydroxylierten Säure, gewählt unter Glycolsäure, Milchsäure, Maleinsäure, Weinsäure, Gluconsäure, Methylmilchsäure, Mandelsäure, Atrolactinsäure, Phenylmilchsäure, Glycerinsäure, Benzylsäure, alpha-Hydroxyhexansäure, alpha-Hydroxyheptansäure, alpha-Hydroxyoctansäure oder einer beta-hydroxylierten Säure, gewählt unter Salicylsäure, Serin; einem Polymer oder Co-Polymer dieser hydroxylierten Säure oder deren Gemischen.

31. Hydroxylierte lipophile Säure nach Anspruch 30, **dadurch gekennzeichnet, dass** die hydrophile, oben genannte hydroxylierte Säure das Reaktionsprodukt einer hydroxylierten Säure ist, die gewählt ist aus der Gruppe, die besteht aus Maleinsäure, Glycolsäure, Gluconsäure, Salicylsäure, Milchsäure, Serin, einem Polymer von Glycolsäure, einem Polymer von Milchsäure, einem Co-Polymer von Glycolsäure und Milchsäure; und einer Verbindung, die die oben genannte Kohlenwasserstoffkette umfasst, gewählt aus der Gruppe, die besteht aus einem Halogenid oder Anhydrid von Stearinsäure, Palmitinsäure, Myristinsäure, Laurinsäure, Undecylensäure, Undecansäure, Ölsäure, Linolsäure, Linolensäure, Essigsäure oder deren Succin- oder Maleinderivaten.

32. Lipophile hydroxylierte Säure nach Anspruch 30 oder 31, **dadurch gekennzeichnet, dass** das Prozentverhältnis zwischen der hydroxylierten Säure und Kohlenwasserstoffkette 0,05 bis 10 und vorzugsweise 0,05 bis 2 Gew.-% ist.

33. Hydroxylierte lipophile Säure nach einem der Ansprüche 30 bis 32, **dadurch gekennzeichnet, dass** die oben genannte lipophile hydroxylierte Säure in trockener Form vorliegt, insbesondere lyophilisiert.

34. Lipophile hydroxylierte Säure nach einem der Ansprüche 30 bis 33, **dadurch gekennzeichnet, dass** die lipophile hydroxylierte Säure im endgültigen Reaktionsmedium mit den Ausgangssäuren vorliegt, die nicht reagiert haben.

35. Lipophile hydroxylierte Säure nach einem der Ansprüche 30 bis 34, **dadurch gekennzeichnet, dass** die lipophile hydroxylierte Fettsäure als einziges Emulsionsmittel verwendet wird oder als Co-Emulsionsmittel.

36. Lipophile hydroxylierte Säure nach einem der Ansprüche 30 bis 35, **dadurch gekennzeichnet, dass** es sich um eine lipophile hydroxylierte Säure handelt, die ein Gemisch von Anhydrid und Ester von Maleinsäure und Stearinsäure umfasst.

37. Lipophile hydroxylierte Säure nach einem der Ansprüche 30 bis 36, **dadurch gekennzeichnet, dass** die lipophile hydroxylierte Säure ein Gemisch von Anhydrid und Ester von Glycolsäure und Palmitinsäure umfasst.

38. Lipophile hydroxylierte Säure nach einem der Ansprüche 30 bis 37, **dadurch gekennzeichnet, dass** die lipophile hydroxylierte Säure ein Gemisch von Anhydrid und Ester von Gluconsäure und Laurinsäure umfasst.

39. Lipophile hydroxylierte Säure nach einem der Ansprüche 30 bis 38, **dadurch gekennzeichnet, dass** die lipophile hydroxylierte Säure ein Gemisch von Anhydrid und Ester von Salicylsäure und Undecylensäure umfasst.

40. Lipophile hydroxylierte Säure nach einem der Ansprüche 30 bis 39, **dadurch gekennzeichnet, dass** die lipophile hydroxylierte Säure ein Gemisch von Anhydrid und Ester von Polymeren von Glycolsäure mit einem Molekulargewicht von 10.000 bis 700.000 Dalton mit einem Gemisch von Chloriden von Öl- und Palmitinsäuren zu 20/80 w/w umfasst.

41. Lipophile hydroxylierte Säure nach einem der Ansprüche 30 bis 40, **dadurch gekennzeichnet, dass** die lipophile hydroxylierte Säure ein Gemisch von Anhydrid und Ester von einem Polymer von Milchsäure mit einem Molekulargewicht von 10.000 bis 700.000 Dalton mit einem Gemisch von Chloriden von Öl- und Palmitinsäuren zu 20/80 w/w umfasst.

42. Lipophile hydroxylierte Säure nach einem der Ansprüche 30 bis 41, **dadurch gekennzeichnet, dass** die lipophile hydroxylierte Säure ein Gemisch von Anhydrid und Ester umfasst, resultierend aus dem Reaktionsprodukt eines Co-Polymers von Glycolsäure und Milchsäure mit einem Molekulargewicht von 10.000 bis 700.000 Dalton mit einem Gemisch von Chloriden von Öl- und Palmitinsäuren zu 20/80 w/w.

43. Lipophile hydroxylierte Säure nach einem der Ansprüche 30 bis 42, **dadurch gekennzeichnet, dass** die lipophile hydroxylierte Säure ein Gemisch von Anhydrid und Ester von Serin und Linol- und Stearinsäure zu 20/80 w/w umfasst.

44. Lipophile hydroxylierte Säure nach einem der Ansprüche 30 bis 43, **dadurch gekennzeichnet, dass** die lipophile hydroxylierte Säure ein Gemisch von Anhydrid und von Ester von Weinsäure und Essigsäure umfasst.

45. Lipophile hydroxylierte Säure nach einem der Ansprüche 30 bis 44, **dadurch gekennzeichnet, dass** die hydroxylierte lipophile Säure ein Gemisch von Anhydrid und Ester von Weinsäure und Bernsteinsäure umfasst.

46. Lipophile hydroxylierte Säure nach einem der Ansprüche 30 bis 45, **dadurch gekennzeichnet, dass** die lipophile hydroxylierte Säure ein Gemisch von Anhydrid und Ester von Weinsäure und Maleinsäure umfasst.

47. Lipophile hydroxylierte Säure nach einem der Ansprüche 30 bis 46, **dadurch gekennzeichnet, dass** die lipophile hydroxylierte Säure ein Gemisch von Anhydrid und Ester von Milchsäure und Stearinsäure umfasst.

48. Lipophile hydroxylierte Säure nach einem der Ansprüche 30 bis 36, **dadurch gekennzeichnet, dass** die hydroxylierte lipophile Säure Amidbindungen durch Reaktion von Maleinsäure mit einem Fettamin umfasst, das durch Laurylamin oder Stearylamin gebildet wird.

49. Lipophile hydroxylierte Säure nach Anspruch 48, **dadurch gekennzeichnet, dass** die hydroxylierte lipophile Säure Amidfunktionen durch Reaktion der Maleinsäure mit Laurylamin umfasst.

50. Lipophile hydroxylierte Säure nach einem der Ansprüche 30 bis 49, **dadurch gekennzeichnet, dass** die lipophile hydroxylierte Säure Anhydridbindungen und Esterbindungen unter Milchsäure und Palmitinsäure umfasst.

51. Kosmetische Zusammensetzung, **dadurch gekennzeichnet, dass** sie als Wirkstoff eine Lipophilsäure, wie in einem der Ansprüche 30 bis 50 definiert, umfasst.

52. Pharmazeutische und/oder dermatologische Zusammensetzung, **dadurch gekennzeichnet, dass** sie eine lipophile hydroxylierte Säure wie in einem der Ansprüche 30 bis 50 definiert umfasst.

53. Zusammensetzung nach Anspruch 51 oder 52, **dadurch gekennzeichnet, dass** sie die lipophile, oben genannte hydroxylierte Säure als Wirkstoff und als Emulgiermittel umfasst.

54. Zusammensetzung nach einem der Ansprüche 51 bis 53, **dadurch gekennzeichnet, dass** der Anteil an lipophiler hydroxylierter Säure zwischen 0,1 und 50 Gew.-% der endgültigen Zusammensetzung, vorzugsweise zwischen 1 und 20 Gew.-% der endgültigen Zusammensetzung liegt.

55. Zusammensetzung, die als Emulgiermittel wenigstens eine lipophile hydroxylierte Säure, wie in einem der Ansprüche 30 bis 50 definiert, umfasst, insbesondere bei einer Konzentration zwischen 0,1 und 50 Gew.-% der endgültigen Zusammensetzung, vorzugsweise zwischen 1 und 20 Gew.-% der endgültigen Zusammensetzung.

56. Zusammensetzung nach Anspruch 55, **dadurch gekennzeichnet, dass** die oben genannte lipophile hydroxylierte Säure als einziges Emulgiermittel oder als Co-Emulsionsmittel verwendet wird.

57. Kosmetisches Verfahren zur keratinolytischen Behandlung von Bereich(en) der Haut, die dessen Bedarf, umfassend die Anwendung auf die Bereiche der Haut einer keratinolytisch effizienten Menge wenigstens einer lipophilen hydroxylierten Säure, wie in einem der Ansprüche 30 bis 50 definiert.

58. Kosmetisches Verfahren, um eine chemische Exfoliation der Haut durchzuführen, um die Hautzellen zu stimulieren, um die Elastizität und die Kohäsion der Haut zu verbessern, um die Haut zu depigmentieren, um die Haut zu befeuchten, um eine Antifalten-Wirkung der Haut, eine Antialterungs-Wirkung auf die Haut, eine Antischuppen-Wirkung auf Bereiche, die aus den Haaren und der Kopfhaut gewählt sind, auszuüben, **dadurch gekennzeichnet, dass** es die Anwendung auf die Haut, das Haar, die Kopfhaut, welche dessen bedarf, einer effizienten Menge einer lipophilen hydroxylierten Säure wie in einem der Ansprüche 30 bis 50 definiert, umfasst.

## Claims

1. Use of a lipophilic hydroxylated acid comprising at least one hydroxyl functional group and at least one acid functional group, **characterised in that** said lipophilic hydroxylated acid comprises a hydrophobic hydrocarbon chain having from 7 to 30 carbon atoms grafted onto the hydroxyl functional group and/or the acid functional group of the lipophilic hydroxylated acid via a covalent bond chosen from the group consisting of an ester bond on the hydroxyl functional group, an anhydride bond on the acid functional group and an amide bond on the acid functional group provided that, in the case of an amide bond, this hydrophobic hydrocarbon chain consists of an alkyl radical having from 10 to 30 carbon atoms resulting from a monoamine, or a combination of these bonds and said hydroxylated acid, before grafting of the hydrophobic hydrocarbon chain, is chosen from the group consisting of an α-hydroxylated acid, chosen from glycolic acid, lactic acid, malic acid, tartaric acid, gluconic acid, methyllactic acid, mandelic acid, atrolactic acid, phenyllactic acid, glyceric acid, benzilic acid, α-hydroxyhexanoic acid, α-hydroxyheptanoic acid, α-hydroxyoctanoic acid; or a β-hydroxylated acid, chosen from serine or a salicylic acid; a polymer or a copolymer of this hydroxylated acid, or their mixtures; as cosmetic agent or as active ingredient;
for the manufacture of a cosmetic composition.

2. Use of a lipophilic hydroxylated acid comprising at least one hydroxyl functional group and at least one acid functional group, **characterised in that** said lipophilic hydroxylated acid comprises a hydrophobic hydrocarbon chain having from 7 to 30 carbon atoms grafted onto the hydroxyl functional group and/or the acid functional group of the lipophilic hydroxylated acid via a covalent bond chosen from the group consisting of an ester bond on the hydroxyl functional group, an anhydride bond on the acid functional group and an amide bond on the acid functional group provided that, in the case of an amide bond, this hydrophobic hydrocarbon chain consists of an alkyl radical having from 10 to 30 carbon atoms resulting from a monoamine, or a combination of these bonds and said hydroxylated acid, before grafting of the hydrophobic hydrocarbon chain, is chosen from the group consisting of an α-hydroxylated acid, chosen from glycolic acid, lactic acid, malic acid, tartaric acid, gluconic acid, methyllactic acid, mandelic acid, atrolactic acid, phenyllactic acid, glyceric acid, benzilic acid, α-hydroxyhexanoic acid, α-hydroxyheptanoic acid, α-hydroxyoctanoic acid; or a β-hydroxylated acid, chosen from serine or a salicylic acid; a polymer or a copolymer of this hydroxylated acid, or their mixtures;
for the manufacture of a dermatological and/or pharmaceutical composition.

3. Use of a lipophilic hydroxylated acid, comprising at least one hydroxyl functional group and at least one acid functional group; **characterised in that** said lipophilic hydroxylated acid comprises a hydrophobic hydrocarbon chain having from 7 to 30 carbon atoms grafted onto the hydroxyl functional group and/or the acid functional group of the lipophilic hydroxylated acid via a covalent bond chosen from the group consisting of an ester bond on the hydroxyl functional group, an anhydride bond on the acid functional group and an amide bond on the acid functional group provided that, in the case of an amide bond, this hydrophobic hydrocarbon chain consists of an alkyl radical having from 10 to 30 carbon atoms resulting from a monoamine, or a combination of these bonds and said hydroxylated acid, before grafting of the hydrophobic hydrocarbon chain, is chosen from the group consisting of an α-hydroxylated acid, chosen from glycolic acid, lactic acid, malic acid, tartaric acid, gluconic acid, citric acid, methyllactic acid, mandelic acid, atrolactic acid, phenyllactic acid, glyceric acid, benzilic acid, α-hydroxybutanoic acid, α-hydroxyhexanoic acid, α-hydroxyheptanoic acid, α-hydroxyoctanoic acid; or a β-hydroxylated acid, chosen from serine or a salicylic acid; a polymer or a copolymer of this hydroxylated acid, or their mixtures;
as emulsifying agent.

4. Use according to one of claims 1 to 3, **characterised in that** the lipophilic hydroxylated acid is the reaction product of a hydroxylated acid chosen from the group consisting of malic acid, glycolic acid, gluconic acid, salicylic acid, lactic acid, serine, a glycolic acid polymer, a lactic acid polymer or a glycolic acid and lactic acid copolymer; and of a compound comprising the hydrophobic hydrocarbon chain chosen from the group consisting of a halide or anhydride of stearic acid, palmitic acid, myristic acid, lauric acid, undecylenic acid, undecanoic acid, oleic acid, linolenic acid, linoleic acid or acetic acid or of their succinic or maleic derivatives.

5. Use according to one of claims 1 to 4, **characterised in that** the ratio by weight of the hydroxylated acid to the hydrophobic chain is 0.05 to 10, and preferably 0.05 to 2.

6. Use according to one of claims 1 to 5, **characterised in that** the lipophilic hydroxylated acid is in a dry form, in particular a lyophilized form.

7. Use according to one of claims 1 to 6, **characterised in that** the lipophilic hydroxylated acid is present in the final reaction medium with the unreacted starting acids.

8. Use according to any one of the preceding claims, **characterised in that** the lipophilic hydroxylated fatty acid is used as sole emulsifying agent or as coemulsifying agent.

9. Use according to one of the preceding claims, **characterised in that** it concerns a lipophilic hydroxylated acid comprising a stearic acid and malic acid ester and anhydride mixture.

10. Use according to one of the preceding claims, **characterised in that** the lipophilic hydroxylated acid comprises a palmitic acid and glycolic acid ester and anhydride mixture.

11. Use according to one of the preceding claims, **characterised in that** the lipophilic hydroxylated acid comprises a lauric acid and gluconic acid ester and anhydride mixture.

12. Use according to one of the preceding claims, **characterised in that** the lipophilic hydroxylated acid comprises an undecylenic acid and salicyclic acid ester and anhydride mixture.

13. Use according to one of the preceding claims, **characterised in that** the lipophilic hydroxylated acid comprises a mixture of anhydrides and esters of glycolic acid polymers of molecular weight of 10,000 to 700,000 Daltons with a palmitic and oleic acid chloride mixture (80/20 w/w).

14. Use according to one of the preceding claims, **characterised in that** the lipophilic hydroxylated acid comprises a mixture of anhydrides and esters of a lactic acid polymer of molecular weight of 10,000 to 700,000 Daltons with a palmitic and oleic acid chloride mixture (80/20 w/w).

15. Use according to one of the preceding claims, **characterised in that** the lipophilic hydroxylated acid comprises a mixture of anhydrides and esters resulting from the reaction product of a glycolic acid and lactic acid copolymer of molecular weight of 10,000 to 700,000 Daltons with a palmitic and oleic acid chloride mixture (80/20 w/w).

16. Use according to one of the preceding claims, **characterised in that** the lipophilic hydroxylated acid comprises a stearic and linoleic acid and serine ester and anhydride mixture (80/20 w/w).

17. Use according to any one of the preceding claims, **characterised in that** the lipophilic hydroxylated acid comprises an acetic acid and tartaric acid ester and anhydride mixture.

18. Use according to any one of the preceding claims, **characterised in that** the lipophilic hydroxylated acid comprises a succinic acid and tartaric acid ester and anhydride mixture.

19. Use according to any one of the preceding claims, **characterised in that** the lipophilic hydroxylated acid comprises a maleic acid and tartaric acid ester and anhydride mixture.

20. Use according to one of the preceding claims, **characterised in that** the lipophilic hydroxylated acid comprises a stearic acid and lactic acid ester and anhydride mixture.

21. Use according to one of the preceding claims, **characterised in that** the lipophilic hydroxylated acid comprises amide bonds by reaction of the maleic acid with a fatty amine consisting of laurylamine or stearylamine.

22. Use according to one of the preceding claims, **characterised in that** the lipophilic hydroxylated acid comprises amide functional groups by reaction of the maleic acid with the laurylamine.

23. Use according to one of the preceding claims, **characterised in that** the lipophilic hydroxylated acid comprises anhydride and ester bonds between the lactic acid and the palmitic acid.

24. Use according to any one of claims 1, 4 to 8, **characterised in that** it concerns the lipophilic malic acid used for an activity chosen from a keratolytic activity, an anti-dandruff activity, an anti-age activity and an anti-wrinkle activity.

25. Use according to one of claims 2, 4 to 8, **characterised in that** it concerns the lipophilic undecylenic acid used for an anti-acne activity.

26. Use according to one of claims 2, 4 to 8, **characterised in that** it concerns the lipophilic salicylic acid used for an anti-acne activity.

27. Use according to one of claims 1, 4 to 8, **characterised in that** it concerns the lipophilic glycolic acid used for carrying out a moisturizing activity which makes it possible to treat dry skins.

28. Use according to any one of claims 1, 4 to 8, **characterised in that** it concerns the lipophilic serine used for a moisturizing activity.

29. Use according to one of claims 2, 4 to 8, **characterised in that** it concerns the lipophilic glycolic and/or salicylic acid used for treating ichthyotic, psoriasis or eczema skins.

30. A lipophilic hydroxylated acid comprising at least one hydroxyl functional group and at least one acid functional group, **characterised in that** said lipophilic hydroxylated acid comprises a hydrophobic hydrocarbon chain having from 7 to 30 carbon atoms grafted onto the hydroxyl functional group and/or the acid functional group of the lipophilic hydroxylated acid via a covalent bond chosen from the group consisting of an ester bond on the hydroxyl functional group, an anhydride bond on the acid functional group and an amide bond on the acid functional group provided that, in the case of an amide bond, this hydrophobic hydrocarbon chain consists of an alkyl radical having from 10 to 30 carbon atoms resulting from a monoamine, or a combination of these bonds and said hydroxylated acid, before grafting of the hydrophobic hydrocarbon chain, is chosen from the group consisting of an α-hydroxylated acid, chosen from glycolic acid, lactic acid, malic acid, tartaric acid, gluconic acid, methyllactic acid, mandelic acid, atrolactic acid, phenyllactic acid, glyceric acid, benzilic acid, α-hydroxyhexanoic acid, α-hydroxyheptanoic acid, α-hydroxyoctanoic acid; or a β-hydroxylated acid, chosen from serine or a salicylic acid; a polymer or a copolymer of this hydroxylated acid, or their mixtures.

31. The lipophilic hydroxylated acid according to claim 30, **characterised in that** the lipophilic hydroxylated acid is the reaction product of a hydroxylated acid chosen from the group consisting of malic acid, glycolic acid, gluconic acid, salicylic acid, lactic acid, serine, a glycolic acid polymer, a lactic acid polymer or a glycolic acid and lactic acid copolymer; and of a compound comprising the hydrophobic hydrocarbon chain chosen from the group consisting of a halide or anhydride of stearic acid, palmitic acid, myristic acid, lauric acid, undecylenic acid, undecanoic acid, oleic acid, linolenic acid, linoleic acid or acetic acid or of their succinic or maleic derivatives.

32. The lipophilic hydroxylated acid according to claim 30 or claim 31, **characterised in that** the ratio by weight of the hydroxylated acid to the hydrophobic chain is 0.05 to 10, and preferably 0.05 to 2.

33. The lipophilic hydroxylated acid according to one of claims 30 to 32, **characterised in that** the lipophilic hydroxylated acid is in a dry form, in particular a lyophilized form.

34. The lipophilic hydroxylated acid according to one of claims 30 to 33, **characterised in that** the lipophilic hydroxylated acid is present in the final reaction medium with the unreacted starting acids.

35. The lipophilic hydroxylated acid according to one of claims 30 to 34, **characterised in that** the lipophilic hydroxylated fatty acid is used as sole emulsifying agent or as coemulsifying agent.

36. The lipophilic hydroxylated acid according to one of claims 30 to 35, **characterised in that** it concerns a lipophilic hydroxylated acid comprising a stearic acid and malic acid ester and anhydride mixture.

37. The lipophilic hydroxylated acid according to one of claims 30 to 36, **characterised in that** the lipophilic hydroxylated acid comprises a palmitic acid and glycolic acid ester and anhydride mixture.

38. The lipophilic hydroxylated acid according to one of claims 30 to 37, **characterised in that** the lipophilic hydroxylated acid comprises a lauric acid and gluconic acid ester and anhydride mixture.

39. The lipophilic hydroxylated acid according to one of claims 30 to 38, **characterised in that** the lipophilic hydroxylated acid comprises an undecylenic acid and salicyclic acid ester and anhydride mixture.

40. The lipophilic hydroxylated acid according to one of claims 30 to 39, **characterised in that** the lipophilic hydroxylated acid comprises a mixture of anhydrides and esters of glycolic acid polymers of molecular weight of 10,000 to 700,000 Daltons with a palmitic and oleic acid chloride mixture (80/20 w/w).

41. The lipophilic hydroxylated acid according to one of claims 30 to 40, **characterised in that** the lipophilic hydroxylated acid comprises a mixture of anhydrides and esters of a lactic acid polymer of molecular weight of 10,000 to 700,000 Daltons with a palmitic and oleic acid chloride mixture (80/20 w/w).

42. The lipophilic hydroxylated acid according to one of claims 30 to 41, **characterised in that** the lipophilic hydroxylated acid comprises a mixture of anhydrides and esters resulting from the reaction product of a glycolic acid and lactic acid copolymer of molecular weight of 10,000 to 700,000 Daltons with a palmitic and oleic acid chloride mixture (80/20 w/w).

43. The lipophilic hydroxylated acid according to one of claims 30 to 42, **characterised in that** the lipophilic hydroxylated acid comprises a stearic and linolenic acid and serine ester and anhydride mixture (80/20 w/w).

44. The lipophilic hydroxylated acid according to any one of claims 30 to 43, **characterised in that** the lipophilic hydroxylated acid comprises an acetic acid and tartaric acid ester and anhydride mixture.

45. The lipophilic hydroxylated acid according to any one of claims 30 to 44, **characterised in that** the lipophilic hydroxylated acid comprises a succinic acid and tartaric acid ester and anhydride mixture.

46. The lipophilic hydroxylated acid according to any one of claims 30 to 45, **characterised in that** the lipophilic hydroxylated acid comprises a maleic acid and tartaric acid ester and anhydride mixture.

47. The lipophilic hydroxylated acid according to one of claims 30 to 46, **characterised in that** the lipophilic hydroxylated acid comprises a stearic acid and lactic acid ester and anhydride mixture.

48. The lipophilic hydroxylated acid according to one of claims 30 to 36, **characterised in that** the lipophilic hydroxylated acid comprises amide bonds by reaction of the maleic acid with a fatty amine consisting of laurylamine or stearylamine.

49. The lipophilic hydroxylated acid according to claim 48, **characterised in that** the lipophilic hydroxylated acid comprises amide functional groups by reaction of the maleic acid with the laurylamine.

50. The lipophilic hydroxylated acid according to one of claims 30 to 49, **characterised in that** the lipophilic hydroxylated acid comprises anhydride and ester bonds between the lactic acid and the palmitic acid.

51. A cosmetic composition, **characterised in that** it comprises a lipophilic hydroxylated acid as active principle, as defined in any one of claims 30 to 50.

52. A pharmaceutical and/or dermatological composition, **characterised in that** it comprises a lipophilic hydroxylated acid, as defined in any one of claims 30 to 50.

53. The composition according to claim 51 or claim 52, **characterised in that** it comprises the lipophilic hydroxylated acid both as active principle and emulsifying agent.

54. The composition according to one of claims 51 to 53, **characterised in that** the proportion of lipophilic hydroxylated acid is between 0.1 % by weight and 50 % by weight of the final composition, preferably between 1 % by weight and 20 % by weight of the final composition.

55. A composition comprising as emulsifying agent at least one lipophilic hydroxylated acid as defined in any one of claims 30 to 50, in particular at a concentration ranging between 0.1 % by weight and 50 % by weight of the final composition, preferably between 1 % by weight and 20 % by weight of the final composition.

56. The composition according to claim 55, **characterised in that** the lipophilic hydroxylated fatty acid is used as sole emulsifying agent or as coemulsifying agent.

57. A cosmetic method for keratolytic treatment on regions of the skin having a need thereof, comprising the application to said regions of the skin of a keratolytic effective amount of at least one lipophilic hydroxylated acid, as defined in any one of claims 30 to 50.

58. A cosmetic method for a chemical exfoliation of the skin, for stimulating the cells of the skin, for improving the elasticity and the cohesion of the skin, for depigmenting the skin, for moisturizing the skin, or for producing an anti-wrinkle effect on the skin, an anti-aging effect on the skin, an anti-dandruff effect, on chosen regions of the hair and of the scalp, **characterised in that** it comprises the application to said skin, to the hair or to the scalp having need thereof, of an effective amount of a lipophilic hydroxylated acid as defined in any one of claims 30 to 50.
